(19) Europäisches Patentamt
European Patent Office
Office européen des brevets

(11) Veröffentlichungsnummer: **0 225 574**
**B1**

(12) # EUROPÄISCHE PATENTSCHRIFT

(45) Veröffentlichungstag der Patentschrift: **16.05.90**

(51) Int. Cl.[5]: **C07D 211/90**, C07D 401/04, C07D 401/12, C07D 413/04, A61K 31/44

(21) Anmeldenummer: **86116657.7**

(22) Anmeldetag: **01.12.86**

(54) **Neue, fluorhaltige 1,4-Dihydropyridine, Verfahren zu ihrer Herstellung sowie ihre Verwendung als Arzneimittel.**

(30) Priorität: **13.12.85 DE 3544211**

(43) Veröffentlichungstag der Anmeldung: **16.06.87 Patentblatt 87/25**

(45) Bekanntmachung des Hinweises auf die Patenterteilung: **16.05.90 Patentblatt 90/20**

(84) Benannte Vertragsstaaten: **AT BE CH DE ES FR GB GR IT LI LU NL SE**

(56) Entgegenhaltungen:
**DE-A- 2 405 658**
**DE-A- 2 841 667**
**DE-A- 3 208 628**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Wehinger, Egbert, Dr., Gellertweg 33, D-5600 Wuppertal 1(DE)**
Erfinder: **Meyer, Horst, Dr., Henselweg 11, D-5600 Wuppertal 1(DE)**
Erfinder: **Knorr, Andreas, Dr., Trillser Graben 10, D-4006 Erkrath 2(DE)**
Erfinder: **Kazda, Stanislav, Dr., Gellertweg 18, D-5600 Wuppertal 1(DE)**

**EP 0 225 574 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 1,4-Dihydropyridin-Derivate, die in ihren Estergruppen fluorierte Kohlenstoffatome enthalten, mehrere Verfahren zu ihrer Herstellung sowie ihre Verwendung als Arzneimittel, insbesondere als kreislaufbeeinflussende Mittel.

Es ist bereits bekannt geworden, daß man 1,4-Dihydro-2,6-dimethyl-4-phenyl-pyridin-3,5-dicarbonsäurediethylester erhält, wenn man 2-Benzylidenacetessigsäureethylester mit β-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1,4-Dihydropyridine interessante pharmakologische Eigenschaften aufweisen (F. Bossert, W. Vater, Naturwissenschaften 58, 578 (1971)).

Weiterhin ist aus den deutschen Offenlegungsschriften 2 841 667 und 3 208 628 bekannt geworden, daß ähnliche Verbindungen als Coronarmittel, antihypertensive Mittel und Mittel zur Steigerung der peripheren Durchblutung verwendet werden können.

Die vorliegende Erfindung betrifft neue 1,4-Dihydropyridin-Derivate der allgemeinen Formel I

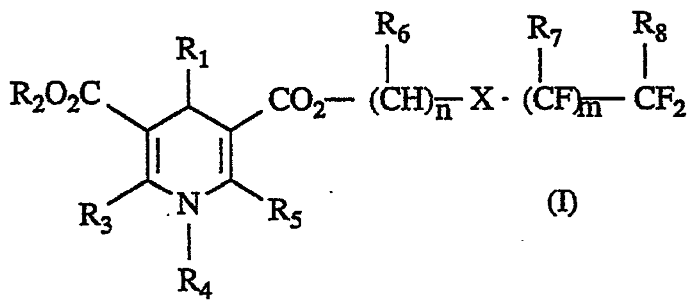

(I)

in welcher

$R_1$ für Phenyl oder Pyridyl steht, welche gegebenenfalls durch 1 oder 2 gleiche oder verschiedene Substituenten aus der Gruppe Chlor, Trifluormethyl, Nitro oder Cyano substituiert sind, oder für Benzoxadiazolyl steht,

$R_2$ einen geradkettigen, verzweigten oder cyclischen Kohlenwasserstoffrest mit bis zu 8 Kohlenstoffatomen darstellt, der gegebenenfalls durch 1 Sauerstoffatom in der Kette unterbrochen ist und/oder der gegebenenfalls substituiert ist durch Fluor, Cyano, Acetoxy, Phenyl, Phenoxy, α-, β- oder γ-Pyridyl oder durch eine Aminogruppe, wobei diese Aminogruppe 2 gleiche oder verschiedene Substituenten aus der Gruppe Alkyl mit bis zu 4 C-Atomen oder Benzyl trägt.

$R_3$ und $R_5$ gleich oder verschieden sind und jeweils für einen geradkettigen oder cyclischen Alkylrest mit bis zu 6 Kohlenstoffatomen stehen, oder

einer der Substituenten $R_3$ oder $R_5$ einen Alkylrest mit bis zu 4 Kohlenstoffatomen darstellt, der durch Acetoxy, Hydroxy, Phthalimido, Amino, Phthalimidoethoxy oder Aminoethoxy substituiert ist, oder

einer der Substituenten $R_3$ oder $R_5$ für die Formyl- oder Nitrilgruppe steht,

$R_4$ für Wasserstoff oder den Morpholinoethylrest steht,

$R_6$ Wasserstoff bedeutet,

$R_7$ und $R_8$ jeweils ein Fluoratom darstellen,

n >/=1 und

m >/=0 ist, wobei die Summe von n und m für eine ganze Zahl von 5 bis 15 steht und

X eine Einfachbindung oder die Gruppe $-N(CH_3)O_2S-$ darstellt,

in Form ihrer racemischen Gemische und ihrer Enantiomeren,

sowie ihre pharmazeutisch unbedenklichen Säureadditionssalze.

Je nach der Art der Substituenten können die erfindungsgemäßen Verbindungen in stereoisomeren Formen existieren, die sich entweder wie Bild und Spiegelbild (Entantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung.

Die Herstellung der erfindungsgemäßen Verbindungen kann nach mehreren Verfahren erfolgen, die sich im wesentlichen an die bekannte Hantzsche 1,4-Dihydropyridin-Synthese anlehnen. Für den Fall, daß in der allgemeinen Formel I die Reste $R_1$, $R_2$, $R_3$, $R_4$, $R_5$, $R_6$, $R_7$, $R_8$ und n, m und X die oben angegebene Bedeutung haben, erfolgt die Herstellung der erfindungsgemäßen Verbindungen dadurch, daß man

A) Yliden-β-ketoester der allgemeinen Formel II

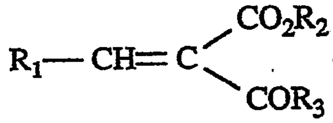

(II)

in welcher

$R_1$, $R_2$ und $R_3$ die oben angegebene Bedeutung haben, mit Enaminocarbonsäureestern der allgemeinen Formel III

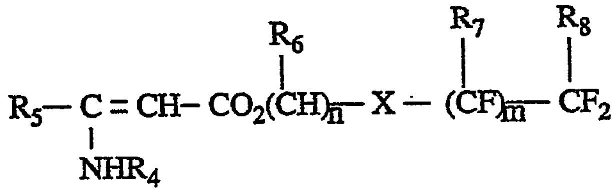

(III)

in welcher

$R_4$, $R_5$, $R_6$, $R_7$, $R_8$, X, n und m die oben angegebene Bedeutung haben, gegebenenfalls in Gegenwart inerter organischer Lösungsmittel umsetzt, oder

B) Yliden-β-ketoester der allgemeinen Formel II

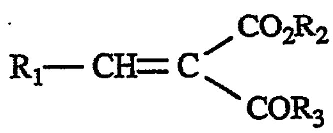

(II)

in welcher

$R_1$, $R_2$ und $R_3$ die oben angegebene Bedeutung haben, mit β-Ketocarbonsäureestern der allgemeinen Formel IV und Aminen der allgemeinen Formel V

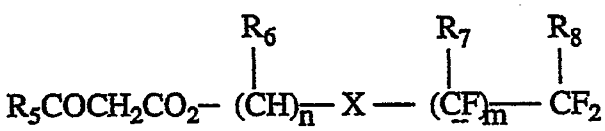

(IV)

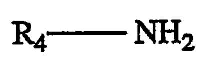

(V)

in welchen

$R_4$, $R_5$, $R_6$, $R_7$, $R_8$, X und n und m die oben angegebene Bedeutung haben, gegebenenfalls in Gegenwart inerter organischer Lösungsmittel umsetzt, oder

C) Yliden-β-ketoester der allgemeinen Formel VI

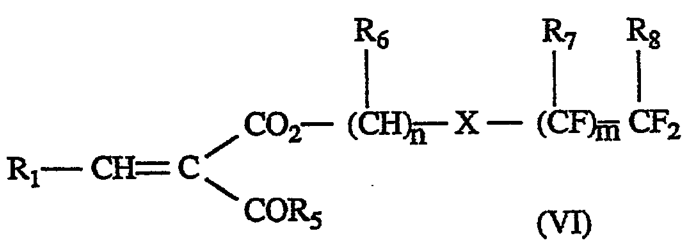

(VI)

in welcher

$R_1$, $R_5$, $R_6$, $R_7$, $R_8$, X, n und m die oben angegebene Bedeutung haben, mit Enaminocarbonsäureestern der allgemeinen Formel VII

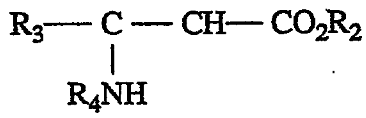 (VII)

in welcher

$R_2$, $R_3$ und $R_4$ die oben angegebene Bedeutung haben, gegebenenfalls in Gegenwart inerter organischer Lösungsmittel umsetzt, oder

D) Yliden-β-ketoester der allgemeinen Formel VI

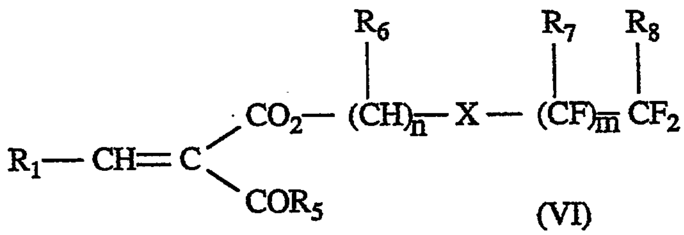

(VI)

in welcher

$R_1$, $R_5$, $R_6$, $R_7$, $R_8$, X, n und m die oben angegebene Bedeutung haben, mit β-Ketocarbonsäureestern der allgemeinen Formel VIII und Aminen der allgemeinen Formel V

$R_3COCH_2CO_2R_2$ (VIII) $\qquad$ $R_4$-$NH_2$ (V)

in welchen

$R_2$, $R_3$ und $R_4$ die oben angegebene Bedeutung haben, gegebenenfalls in Gegenwart inerter organischer Lösungsmittel umsetzt, oder

E) Aldehyde der allgemeinen Formel IX

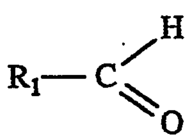 (IX)

in welcher

$R_1$ die oben angegebene Bedeutung hat, mit Enaminocarbonsäureestern der allgemeinen Formel II und β-Ketocarbonsäureestern der allgemeinen Formel VIII

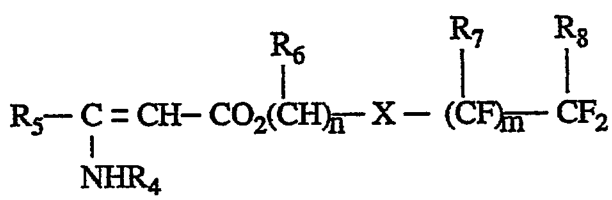 (III)

$R_3COCH_2CO_2R_2$ (VIII)

in welchen

$R_2$, $R_3$, $R_4$, $R_5$, $R_6$, $R_7$, $R_8$, X, n und m die oben angegebene Bedeutung haben, gegebenenfalls in Gegenwart inerter organischer Lösungsmittel zur Reaktion bringt, oder

F) Aldehyde der allgemeinen Formel IX

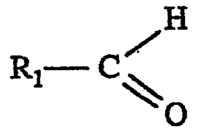 (IX)

in welcher

$R_1$ die oben angegebene Bedeutung hat, mit Enaminocarbonsäureestern der allgemeinen Formel VII und β-Ketocarbonsäureestern der allgemeinen Formel IV

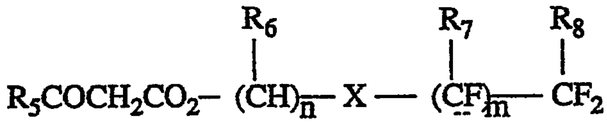

(IV)

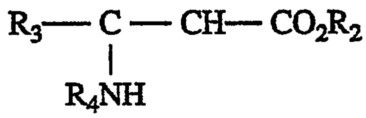 (VII)

in welchen

$R_2$, $R_3$, $R_4$, $R_5$, $R_6$, $R_7$, $R_8$, X, n und m die oben angegebene Bedeutung haben, gegebenenfalls in Gegenwart inerter organischer Lösungsmittel umsetzt, oder

G) die 1,4-Dihydropyridinmonocarbonsäuren der allgemeinen Formeln X bzw. XI

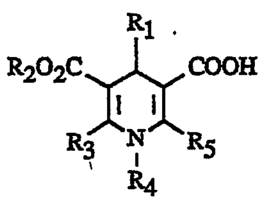

(X)

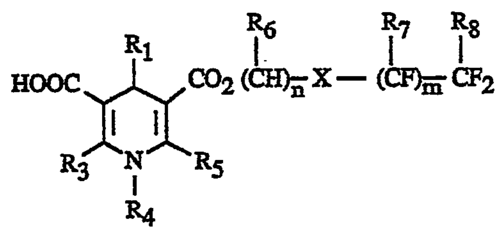

(XI)

in welchen

$R_1$, $R_2$, $R_3$, $R_4$, $R_5$, $R_6$, $R_7$, $R_8$, X, n und m die oben angegebene Bedeutung haben, nach üblichen Methoden der Veresterung von Carbonsäuren (z.B. über das Säurechlorid oder -imidazolid oder in Gegenwart von Dicyclohexylcarbodiimid) mit Alkoholen der allgemeinen Formel XII bzw. XIII

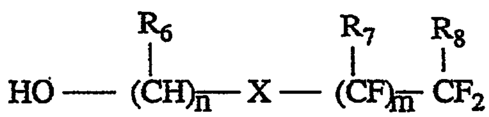

$R_2$— OH

(XII) (XIII)

in welchen

$R_2$, $R_6$, $R_7$, $R_8$, X, n und m die oben angegebene Bedeutung haben, verestert.

Verwendet man optisch aktive enantiomerenreine 1,4-Dihydropyridinmonocarbonsäuren der allgemeinen Formel X bzw. XI, in denen die jeweiligen C4-Kohlenstoffatome des Dihydropyridinrings die alleinigen Chiralitätszentren darstellen, so erhält man durch Veresterung mit achiralen Alkoholen der Formeln XII bzw. XIII und unter der Voraussetzung, daß die Estergruppen in den Endprodukten verschieden sind, optisch aktive enantiomerenreine Verbindungen der allgemeinen Formel I mit achiralen Substituenten. Naturgemäß lassen sich auf diesem Wege je nach der Wahl der optisch aktiven Ausgangssäuren X bzw. XI und der Alkoholkomponenten XII und XIII erfindungsgemäße Verbindungen mit zusätzlichen (einheitlich konfigurierten) Chiralitätszentren erhalten.

H) Für den Fall, daß in der allgemeinen Formel I die Reste $R_1$, $R_2$, $R_4$, $R_6$, $R_7$, $R_8$ und n, m und X die oben angegebene Bedeutung haben und $R_3$ oder $R_5$ für die Formyl- oder die Nitrilgruppe oder für einen Alkylrest stehen, der durch Hydroxy, Amino oder Aminoalkoxy substituiert ist, erfolgt die Herstellung der erfindungsgemäßen Verbindungen vorzugsweise derart, daß man nach den Verfahren A) bis G) geeignete erfindungsgemäße Zwischenprodukte synthetisiert und diese in Folgereaktionen weiter umsetzt. Stehen $R_3$ oder $R_5$ beispielsweise für eine Formylgruppe, so erhält man diese Derivate durch saure Hydrolyse erfindungsgemäßer Verbindungen der allgemeinen Formel I, in der $R_3$ oder $R_5$ gleich Formyl lassen sich durch Umsetzung mit Hydroxylamin und anschließende Dehydratisierung des entstandenen Oxims nach literaturbekannten Methoden in die erfindungsgemäßen Stoffe der allgemeinen Formel I mit $R_3$ oder $R_5$ gleich Nitril überführen.

Auf ähnliche Weise erhält man die erfindungsgemäßen Verbindungen der allgemeinen Formel I, in welcher $R_3$ oder $R_5$ für einen Alkylrest steht, der durch a) Hydroxy, b) Amino oder c) Aminoalkoxy substituiert ist, indem man a) die entsprechenden erfindungsgemäßen Acyloxyderivate sauer oder alkalisch hydrolysiert, b) die entsprechenden nach oben beschriebenen Verfahren herstellbaren Phthalimido-Verbindungen beispielsweise hydrazinolysiert oder c) die entsprechenden erfindungsgemäßen Phthalimidoalkoxyderivate mit Hydrazin umsetzt.

Die erfindungsgemäßen Verbindungen besitzen wertvolle pharmakologische Eigenschaften. Insbesondere aufgrund ihrer starken und langanhaltenden kreislaufbeeinflussenden Wirkung können sie als antihypertensive Mittel, als Vasodilatatoren, als Cerebraltherapeutika sowie als Coronartherapeutika Verwendung finden und sind somit als Bereicherung der Pharmazie anzusehen.

Von besonderem Interesse ist die starke und langanhaltende blutdrucksenkende Wirkung. Je nach Art der verwendeten Ausgangsstoffe können die Synthesevarianten für die erfindungsgemäßen Verbindungen durch folgende Formelschemata wiedergegeben werden:

A)

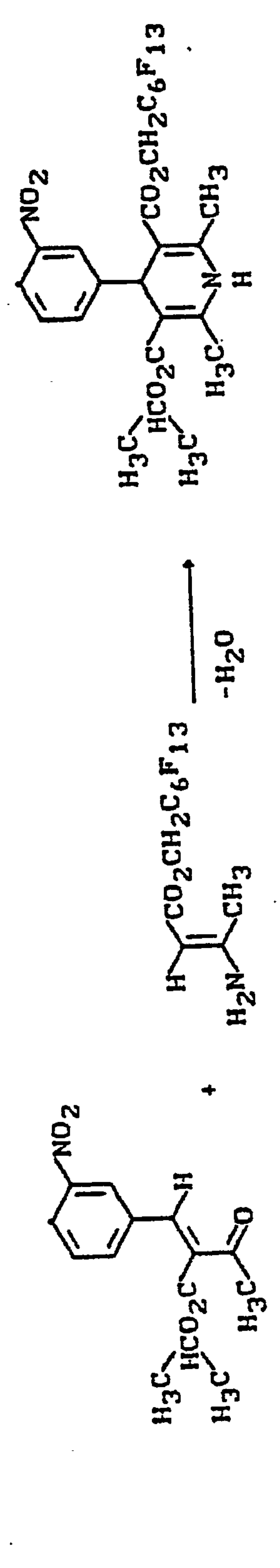


B)

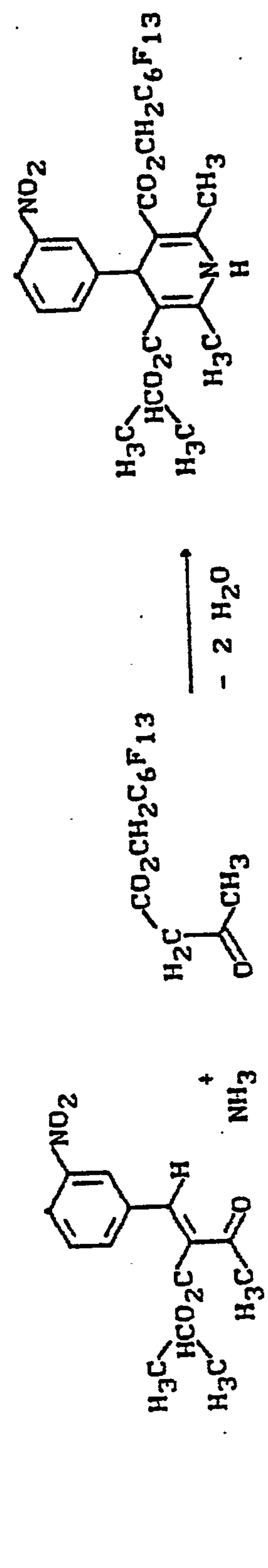


C)

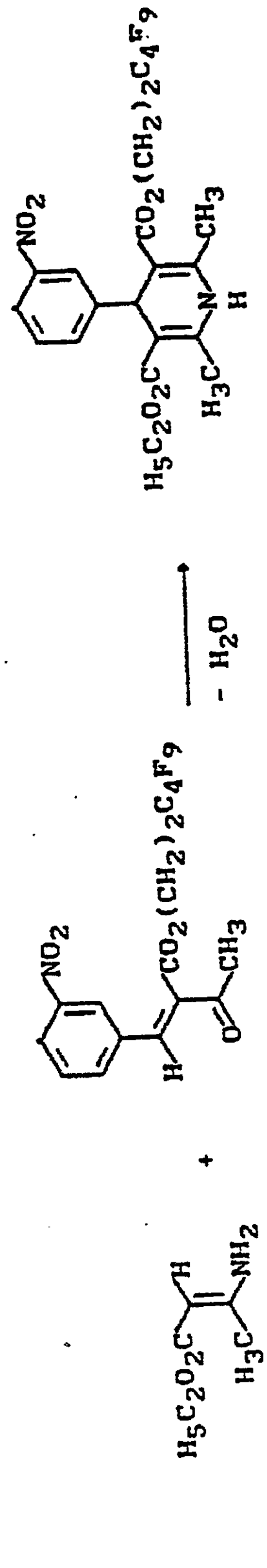

D)

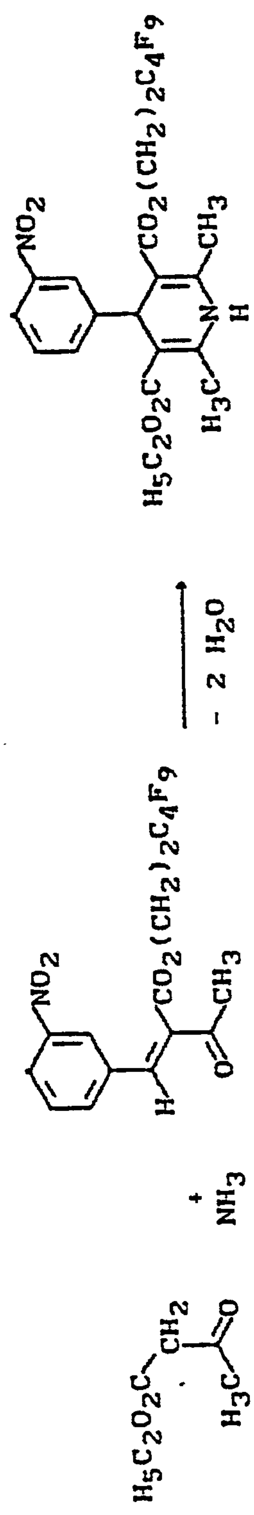


E)

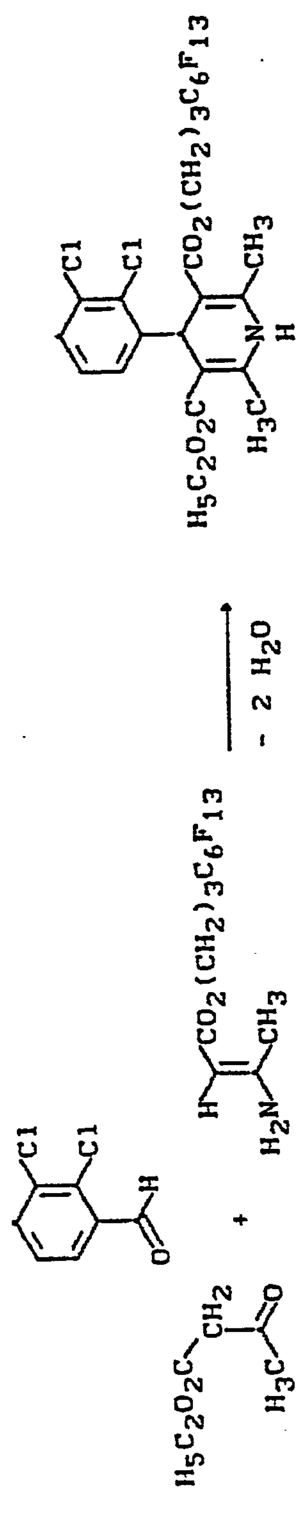


F)

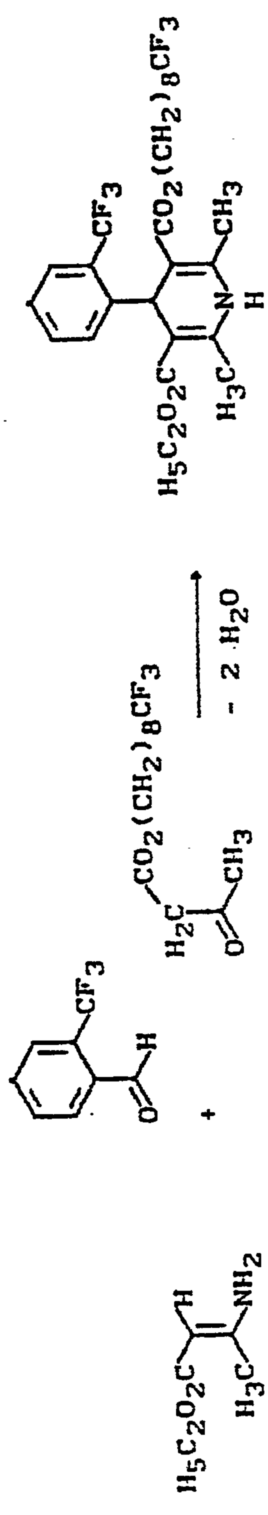

G)

H)

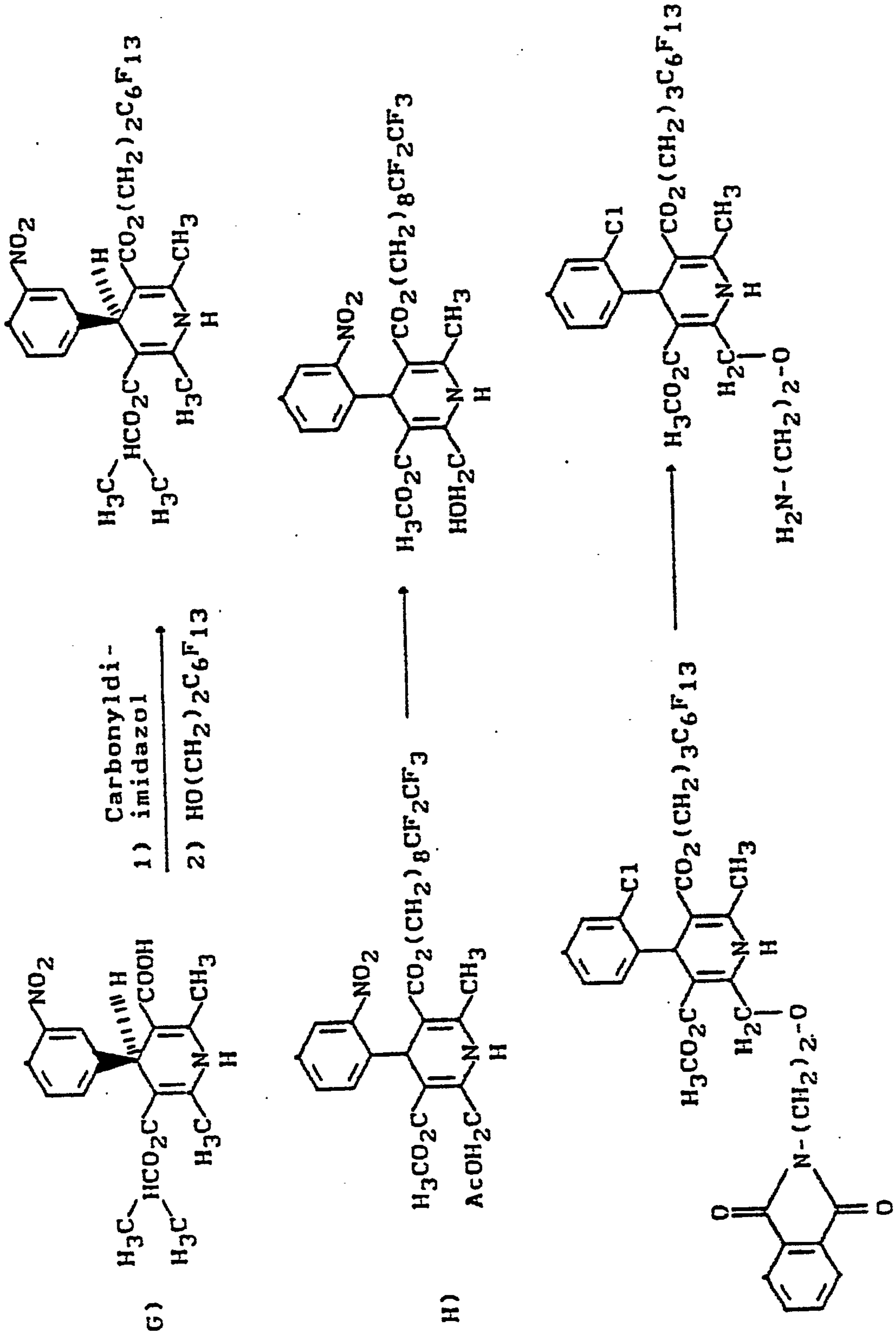

Verfahrensvarianten A und C

Gemäß Verfahren A und C wird ein Yliden-β-ketoester der allgemeinen Formel II bzw. VI

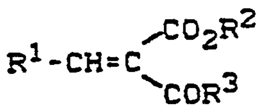

II

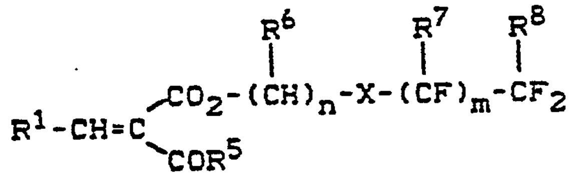

VI

mit einem Enaminocarbonsäureester der allgemeinen Formel III bzw. Formel VII

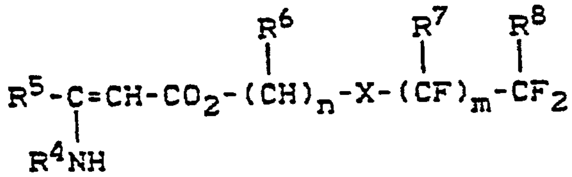

III

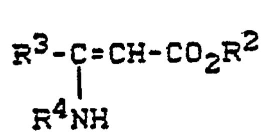

VII

zur Reaktion gebracht.

Die als Ausgangsstoffe verwendeten Yliden-β-ketoester der allgemeinen Formel II bzw. VI sind literaturbekannt oder können nach literaturbekannten Methoden hergestellt werden (vgl. z.B. G. Jones "The Knoevenagel Condensation" in Org. Reactions, Vol. XV, 204ff. (1969)).

Als Beispiele seien genannt:
Benzylidenformylessigsäuremethylester,
Benzylidenacetessigsäuremethylester,
2'-Nitrobenzylidenacetessigsäurethylester,
3'-Nitrobenzylidenacetessigsäure-n-butylester,
3'-Nitrobenzylidenacetessigsäureisobutylester,
3'-Nitrobenzylidenacetessigsäuredodecylester,
3'-Nitrobenzylidenacetessigsäurecyclopentylester,
2'-Chlorbenzylidenacetessigsäureallylester,
2',3'-Dichlorbenzylidenacetessigsäurebenzylester,
2'-Chlorbenzylidenacetessigsäure-(2-methoxyethyl)-ester,
3'-Chlorbenzylidenacetessigsäure-(2-propoxyethyl)-ester,
3'-Cyanobenzylidenacetessigsäure-(2-chlorethyl)-ester,
2'-Chlorbenzylidenacetessigsäure-(2,2,2-trifluorethyl)-ester,
2'-Trifluormethylbenzylidenacetessigsäure-(2-cyanoethyl)-ester,
2'-Difluormethoxybenzylidenacetessigsäure-(2-acetoxyethyl)-ester,
3'-Methylsulfonylbenzylidenacetessigsäure-(2-phenoxyethyl)-ester,
3'-Nitrobenzylidenacetessigsäure-(2-N-benzyl-N-methylaminoethyl)-ester,
3'-Nitrobenzylidenacetessigsäure-(2-(3-pyridyl)-ethyl)-ester,
3'-Nitrobenzylidenpropionylessigsäurepropylester,
4-Acetoxy-2-(3'-nitrobenzyliden)-acetessigsäuremethylester,
4-(2-Phthalimidoethoxy)-2-(2'-chlorbenzyliden)-acetessigsäureethylester,
3'-Nitrobenzylidenacetessigsäure-(2-perfluorbutyl-ethyl)-ester,
3'-Chlorbenzylidenacetessigsäure-(2-perfluorhexyl-ethyl)-ester,
2'-Nitrobenzylidenacetessigsäure-(3-perfluorhexyl-propyl)-ester,
2'-Cyanobenzylidenacetessigsäure-(9,9,9-trifluornonyl)-ester,
2'-Trifluormethylbenzylidenacetessigsäure-perfluorhexylmethyl-ester,
2',3'-Dichlorbenzylidenacetessigsäure-(2-perfluorbutylethyl)-ester,
2'-Chlor-3'-nitrobenzylidenacetessigsäure-(3-perfluorhexyl-propyl)-ester,
α-Acetyl-β-(pyridyl-3)-acrylsäure-perfluorhexylmethyl-ester,
α-Acetyl-β-(pyridyl-2)-acrylsäure-(3-perfluorhexyl-propyl)-ester,
α-Acetyl-β-(benzoxadiazolyl-4)-acrylsäure-(2-perfluorbutyl-ethyl)-ester.

Die als Ausgangsstoffe verwendeten Enaminocarbonsäureester der allgemeinen Formel III bzw. VII sind literaturbekannt oder können nach literaturbekannten Methoden hergestellt werden (vgl. z.B. A.C. Cope, J. Am. Chem. Soc. 67, 1017 (1945)).

Als Beispiele seien genannt:
3-Aminocrotonsäuremethylester, 3-Aminocrotonsäurebutylester, 3-Aminocrotonsäureheptylester, 3-Aminocrotonsäureisopropylester, 3-Aminocrotonsäurepentylester, 3-Aminocrotonsäurecyclopentylester, 3-Aminocrotonsäure-(2,2,2-trifluorethyl)-ester, 3-Aminocrotonsäure-(2-cyanoethyl)-ester, 3-Aminocrotonsäure-(2-acetoxyethyl)-ester, 3-Aminocrotonsäure-(2-propoxyethyl)-ester, 3-Aminocrotonsäure-(2-phenoxyethyl)-ester, 3-Aminocrotonsäurebenzylester, 3-Aminocrotonsäure-(2-dimethylaminoethyl)-ester, 3-Aminocrotonsäure-(2-(N-benzyl-N-methyl)-aminoethyl)-ester, 3-Aminocrotonsäure-(9,9,9,-trifluornonyl)-ester, 3-Aminocrotonsäure-(3-prefluorhexyl-propyl)-ester, 3-Aminocrotonsäure-perfluorhexylmethyl-ester, 3-Aminocrotonsäure-(2-perfluorbutyl-ethyl)-ester.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether, oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise zwischen 20 und 100° C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei den Durchführung der erfindungsgemäßen Verfahren wird ein Mol des Yliden-β-ketoesters der Formel II bzw. VI mit einem Mol Enaminocarbonsäureester der Formel III bzw. VII in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den gegebenenfalls erst nach Eiskühlung kristallin erhaltenen Rückstand aus einem geeigneten Lösungsmittel umkristallisiert.

Verfahrensvarianten B und D

Gemäß Verfahren B und D wird ein Yliden-β-ketoester der allgemeinen Formel II bzw. VI

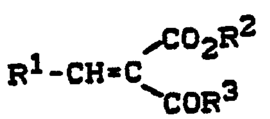

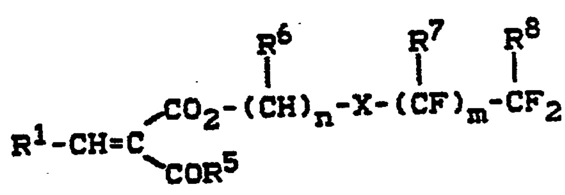

II VI

mit einem β-Ketocarbonsäureester der allgemeinen Formel IV bzw. VIII

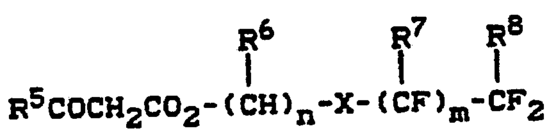

IV

$$R^3COCH_2CO_2R^2$$

VIII

und einem Amin der allgemienen Formel V

$$R^4\text{-}NH_2 \quad V$$

zur Reaktion gebracht.

Beispiele der als Ausgangskomponenten verwendeten Yliden-β-ketoester der Formel II bzw. VI sind bereits unter den Verfahrensvarianten A und C aufgeführt.

Die als Ausgangsstoffe verwendeten β-Ketocarbonsäureester der Formel IV bzw. VIII sind literaturbekannt oder können nach literaturbekannten Methoden hergestellt werden (vgl. z.B. D. Borrmann, "Umsetzung von Diketen mit Alkoholen, Phenolen und Mercaptanen", in Houben-Weyl, Methoden der Organischen Chemie, Vol. VII/4, 230 ff (1968); Y. Oikawa, K. Sugano und O. Yonemitsu, J. Org. Chem. 43, 2087 (1978)).

Als Beispiele seien genannt:
Acetessigsäuremethylester, Acetessigsäureheptylester, Acetessigsäureisopropylester, Acetessigsäureneopentylester, Acetessigsäurecyclopentylester, Acetessigsäure-(2-methoxyethyl)-ester, Acetessigsäure-(2,2,2-trifluorethyl)-ester, Acetessigsäure-(2-cyanoethyl)-ester, Acetessigsäure-(2-acetoxyethyl)-ester, Acetessigsäurebenzylester, Acetessigsäure-(2-phenoxyethyl)-ester, Acetessigsäure-(2-N-benzyl-N-methyl-aminoethyl)-ester, Acetessigsäure(9,9,9-trifluornonyl)-ester, Acetessigsäure-(3-perfluorhexyl-propyl)-ester, Acetessigsäure-(2-perfluorbutylethyl)-ester.

Die erfindungsgemäß verwendbaren Amine der Formel V sind bereits bekannt.

Als Beispiele seien genannt:

Ammoniak, Methylamin, n-Propylamin, β-Methoxyethylamin, Benzylamin, 2-(N-Morpholino)-ethylamin.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung der erfindungsgemäßen Verfahren werden die an der Reaktion beteiligten Stoffe der Formeln II bzw. VI, IV bzw. VIII und V jeweils in molaren Mengen eingesetzt. Das verwendete Amin wird zweckmäßigerweise im Überschuß von 1 bis 2 Mol zugegeben. Die erfindungsgemäßen Verbindungen können leicht durch Umkristallisation aus einem geeigneten Lösungsmittel gereinigt werden.

Verfahrensvariante E und F

Gemäß Verfahren E und F wird ein Aldehyd der allgemeinen Formel IX

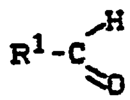 IX

entweder (Verfahren E) mit einem Enaminocarbonsäureester der Formel III und einem β-Ketocarbonsäureester der Formel VIII

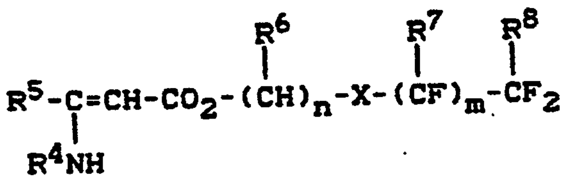

III

$R^3COCH_2CO_2R^2$

VIII

oder (Verfahren F) mit einem Enaminocarbonsäureester der Formel VII

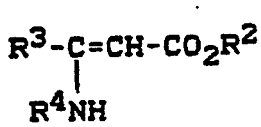

VII

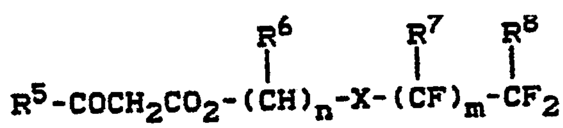

IV

und einem β-Ketocarbonsäureester der allgemeinen Formel IV zur Reaktion gebracht.

Die erfindungsgemäß verwendbaren Enaminocarbonsäureester der allgemeinen Formel III und VII sind bereits unter den Verfahrensvarianten A und C angegeben. Die erfindungsgemäß einsetzbaren β-Ketocarbonsäureester der allgemeinen Formel VIII und IV wurden bereits unter den Verfahrensvarianten B und D beschrieben.

Die als Ausgangsstoffe verwendbaren Aldehyde der Formel IX sind literaturbekannt oder können nach literaturbekannten Methoden hergestellt werden (vgl. z.B. E. Mosettig, Org. Reactions VIII, 281 ff (1954)).

Als Beispiele seien genannt:

Benzaldehyd, 2- oder 3-Phenylbenzaldehyd, 2-Methylbenzaldehyd, 2- oder 3-Isopropylbenzaldehyd, 2- oder 3-Cyclopropylbenzaldehyd, 2,3-Tetramethylenbenzaldehyd, 3,4-Dioxymethylenbenzaldehyd, 3-Methoxybenzaldehyd, 2,- 3- oder 4-Chlor/Brom/Fluorbenzaldehyd, 2- oder 3-Trifluormethylbenzaldehyd, 2-, 3- oder 4-Trifluormethoxybenzaldehyd, 2-Difluormethoxybenzaldehyd, 2- oder 3-Nitrobenzaldehyd, 2- oder 3-Cyanobenzaldehyd, 3-Azidobenzaldehyd, 2- oder 3-Methylthiobenzaldehyd, 3-Methylsulfinylbenzaldehyd, 2-Methylsulfonylbenzaldehyd, 2,3-Dichlorbenzaldehyd, 2-Fluor-3-chlorbenzaldehyd, 2-Chlor-3-trifluormethylbenzaldehyd, 3-Chlor-2-trifluormethylbenzaldehyd, 2,4-Dinitrobenzaldehyd, 2-Chlor-6-nitrobenzaldehyd, 4-Chlor-2-nitrobenzaldehyd, Thiophen-2-aldehyd, Furan-2-aldehyd, Pyrrol-2-aldehyd, Pyrazol-4-aldehyd, Imidazol-2-aldehyd, Oxazol-2-aldehyd, Isoxazol-3-aldehyd, Thiazol-2-aldehyd, Pyridin-2- oder-3-aldehyd, 6-Methyl-pyridin-2-aldehyd, 2-Methylthio-pyridin-3-aldehyd, Indol-3-aldehyd, Benzimidazol-2-aldehyd, Benzoxazol-4-aldehyd, Benzoxadiazol-4-aldehyd, Chinolin-4-aldehyd, Chinazolin-2-aldehyd, Chinoxalin-5-aldehyd.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise jedoch bei der Siedetemperatur des jeweilgen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe jeweils in molaren Mengen eingesetzt.

Verfahrensvariante G

Gemäß Verfahren G wird eine racemische oder enantiomerenreine 1,4-Dihydropyridinomonocarbonsäure der allgemeinen Formel X oder XI

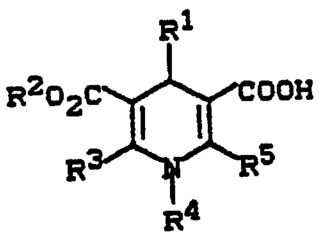

X

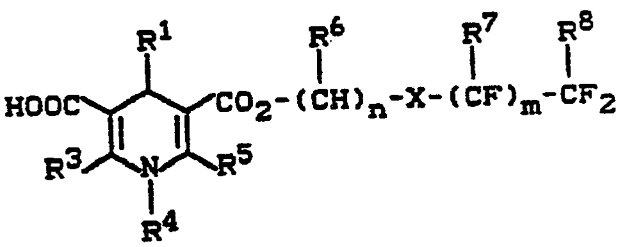

XI

mit einem Alkohol der allgemeinen Formel XII bzw. XIII

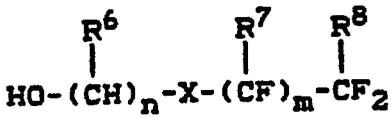

XII

$R^2$-OH

XIII

verestert.

Die erfindungsgemäß verwendbaren racemischen oder enantiomerenreinen 1,4-Dihydropyridinmonocarbonsäuren der allgemeinen Formel X sind literaturbekannt oder können nach literaturbekannten Methoden hergestellt werden (vgl. z.B. DOS 2 921 429; T. Shibanuma, M. Iwanami, K.Okuda, T. Takenaka und M. Murakami, Chem. Pharm. Bull. 28, 2809 (1980)).

Als Beispiele seien genannt:

1,4-Dihydro-2,6-dimethyl-4-(3-nitrophenyl)-pyridin-3,5-dicarbonsäuremonomethylester
1,4-Dihydro-2,6-dimethyl-4-(3-nitrophenyl)-pyridin-3,5-dicarbonsäuremonoisopropylester
1,4-Dihydro-2,6-dimethyl-4-(2-nitrophenyl)-pyridin-3,5-dicarbonsäuremonoisobutylester
1,4-Dihydro-2,6-dimethyl-4-(2-chlorphenyl)-pyridin-3,5-dicarbonsäuremonomethylester
1,4-Dihydro-2,6-dimethyl-4-(2,3-dichlorphenyl)-pyridin-3,5-dicarbonsäuremonomethylester

1,4-Dihydro-2,6-dimethyl-4-(2-trifluormethylphenyl)-pyridin-3,5-dicarbonsäuremonoethylester
1,4-Dihydro-2,6-dimethyl-4-(2,1,3-benzoxadiazolyl-4-)-pyridin-3,5-dicarbonsäuremonoisopropylester
1,4-Dihydro-2,6-dimethyl-4-(3-nitrophenyl)-pyridin-3,5-dicarbonsäuremono-(2-perfluorbutyl-ethyl)-ester
1,4-Dihydro-2,6-dimethyl-4-(3-nitrophenyl)-pyridin-3,5-dicarbonsäuremono-(2-perfluorhexyl-ethyl)-ester
1,4-Dihydro-2,6-dimethyl-4-(3-nitrophenyl)-pyridin-3,5-dicarbonsäuremono-(3-perfluorhexyl-propyl)-ester
1,4-Dihydro-2,6-dimethyl-4-(3-nitrophenyl)-pyridin-3,5-dicarbonsäuremono-(9,9,9-trifluornonyl)-ester.

Die erfindungsgemäß verwendbaren Alkohole sind literaturbekannt oder können nach literaturbekannten Methoden hergestellt werden (vgl. z.B. N.O. Brace, L.W. Marshall, C.J. Pinson und G. van Wingerden, J. Org. Chem. 49, 2361 (1984)).

Als Beispiel seien genannt:
Methanol, Ethanol, Butanol, Isopropanol, Benzylalkohol, 2-Methoxyethanol, 2-Dimethylaminoethanol, 2-(N-Benzyl-N-methyl)-aminoethanol, 2-Perfluorbutylethanol, Perfluorhexylmethanol, 3-Perfluorhexylpropanol, 9,9,9-Trifluornonanol.

Die Durchführung des erfindungsgemäßen Verfahrens lehnt sich an die literaturbekannte Methodik zur Veresterung von Carbonsäuren an. Dabei wird die Carbonsäure zunächst in eine aktivierte Form wie z.B. das Säurechlorid oder das Imidazolid überführt, die entweder als solche isoliert und in einem zweiten Reatkionsschritt umgesetzt wird oder die in situ direkt zu den erfindungsgemäßen Verbindungen alkanolisiert wird. Als aktivierende Agentien seien neben den anorganischen Halogeniden wie Thionylchlorid, Phosphortrichlorid oder Phosphorpentachlorid oder dem Carbonyldiimidazol, Carbodiimide wie Dicyclohexylcarbodiimid oder 1-Cyclohexyl-3-[2-(N-methyl-morpholino)-ethyl]-carbodiimid-p-toluolsulfonat oder N-Hydroxyphthalimid oder N-Hydroxy-benzotriazol in Gegenwart von Dicyclohexylcarbodiimid beispielhaft erwähnt. Naturgemäß lassen sich die 1,4-Dihydropyridinmonocarbonsäuren auch in Salze überführen, die mit Substraten der allgemeinen Formel XIV bzw. XV,

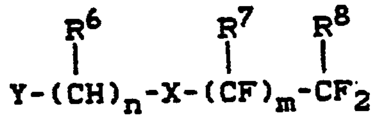

XIV

$R^2-Y$

XV

in welchen Y eine nucleofuge Gruppe wie beispielsweise Iodid oder Tosylat darstellt, zu den erfindungsgemäßen Verbindungen umgesetzt werden können.

Als Verdünnungsmittel kommen alle inerten organischen Lösungsmittel in Frage. Hierzu gehören vorzugsweise Ether wie Dioxan, Tetrahydrofuran, Glykolmonomethylether oder Glykoldimethylether, halogenierte Kohlenwasserstoffe wie Dichlormethan oder Trichlormethan, Dimethylformamid, Dimethylsulfoxid, Acetonitril, Pyridin und Hexamethylphosphorsäuretriamid. Isoliert man die aktivierten Zwischenstufen der 1,4-Dihydropyridinmonocarbonsäuren, so können die Alkohole der Formel XII bzw. XIII auch allein als Verdünnungsmittel verwendet werden.

Die Alkanolyse wird zweckmäßigerweise durch Zugabe katalytischer oder molarer Mengen eines basischen Hilfsstoffs beschleunigt.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Verfahrensvariante H

Für den Fall, daß $R^3$ oder $R^5$ in der allgemeinen Formel I für eine Formylgruppe steht, erfolgt die Herstellung dieser erfindungsgemäßen Verbindungen vorzugsweise durch saure Hydrolyse der erfindungsgemäßen Verbindungen der allgemeinen Formel I, in welcher $R^3$ oder $R^5$ einen Dialkoxymethylrest mit 1 bis 2 Kohlenstoffatomen je Alkoxygruppe bedeutet.

Steht $R^3$ oder $R^5$ in der allgemeinen Formel I für eine Nitrilgruppe, so erhält man diese Verbindungen vorzugsweise durch Umsetzung der erfindungsgemäßen Verbindungen der allgemeinen Formel I, in welcher $R^3$ oder $R^5$ für eine Formylgruppe steht, mit Hydroxylamin und anschließende Dehydratisierung des entstandenen Oxims, beispielsweise durch Erhitzen in einem Gemisch von Acetanhydrid und Eisessig.

Steht $R^3$ oder $R^5$ in der allgemeinen Formel I für einen Alkylrest, der durch eine Hydroxygruppe substituiert ist, so erhält man diese Substanzen durch Hydrolyse der entsprechenden, erfindungsgemäßen Acetoxyderivate unter sauren oder alkalischen Bedingungen.

Für den Fall, daß $R^3$ oder $R^5$ in der allgemeinen Formel I einen Alkylrest darstellt, der durch eine Amino- oder eine Aminoalkoxygruppe substituiert ist, so erfolgt die Darstellung dieser erfindungsgemäßen Substanzen beispielsweise dadurch, daß man die entsprechenden, erfindungsgemäßen Phthalimido- bzw. Phthalimidoalkoxy-Verbindungen gemäß dem Stand der Technik mit Hydrazin umsetzt.

Die vorstehenden Herstellungsverfahren sind lediglich zur Verdeutlichung angegeben, und die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Die neuen Verbindungen haben ein breites und vielseitiges pharmakologisches Wirkungsspektrum.

Im einzelnen konnten im Tierexperiment folgende Haupt-Wirkung nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritänlilchen herzentlastenden Effekt verstärkt. Sie beeinflussen bzw. verändern den Herzstoffwechsel im Sinne einer Energieersparnis.
2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten manifestieren.
4. Die Verbindungen senken langanhaltend den Blutdruck von normotonen und hypertonen Tieren und können somit als antihypertensive Mittel verwendet werden.
5. Die Verbindungen haben stark muskulär-spasmolytische Wirkungen, die an der glatten Muskulatur des Magens, Darmtrakis, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheiten im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfslösungsmittel seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den obengenannten Hilfsstoffen mit verschiedenen Geschmacksaufbesseren oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,005 bis 5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,02 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann

es in einigen Fällen ausreichend sein, mit weniger als der vorgenannten Mindestmenge auszukommen, während in anderen Fällen die genannte obere Grenze überschritten werden muß.

Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

Beispiel 1 (Verfahrensvariante A)

1,4-Dihydro-2,6-dimethyl-4-(3-nitrophenyl)-pyridin-3,5-dicarbonsäure-methyl-(4-perfluorethyl-butyl)-ester

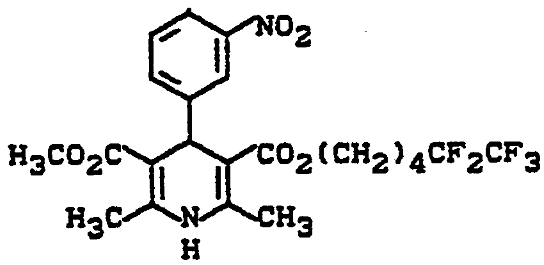

8,69 g (35 mmol) 2-(3-Nitrobenzyliden)-acetessigsäuremethylester wurden zusammen mit 9,63 g (35 mmol) 3-Aminocrotonsäure-(4-perfluorethyl-butyl)-ester in 150 ml Isopropanol aufgenommen und 15 Stunden unter Rückfluß erhitzt. Anschließend wurde das Lösungsmittel unter vermindertem Druck abdestilliert und der ölige Rückstand durch Verreiben mit wenig Ether zur Kristallisation gebracht. Das Rohprodukt wurde abgesaugt und aus Ethanol umkristallisiert.
Fp.: 119-121° C
Ausbeute: 13,3 g (75 % der Theorie).

Beispiel 2 (Verfahrensvariante C)

1,4-Dihydro-2,6-dimethyl-4-(3-nitrophenyl)-pyridin-3,5-dicarbonsäure-methyl-(2-perfluorbutyl-ethyl)-ester

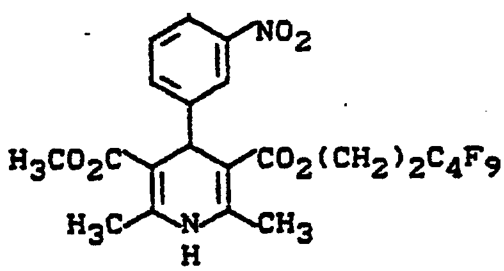

Eine Lösung von 33,69 g (70 mmol) 2-(3-Nitrobenzyliden)-acetessigsäure-(2-perfluorbutyl-ethyl)-ester und 8,06 g (70 mmol) 3-Aminocarbonsäuremethylester in 200 ml Ethanol wurde 15 Stunden zum Sieden erhitzt. Anschließend wurde das Lösungsmittel unter Vakuum abdestilliert und der feste Rückstand säulenchromatographisch über Kieselgel mit einem Gemisch aus Toluol/Aceton = 5/1 als Eluierungsmittel gereinigt.
Fp.: 106° C
Ausbeute: 28,7 g (71 % der Theorie).

Beispiel 3 (Verfahrensvariante G)

1,4-Dihydro-2,6-dimethyl-4-(3-nitrophenyl)-pyridin-3,5-dicarbonsäure-isopropyl-(4-perfluorethyl-butyl)-ester

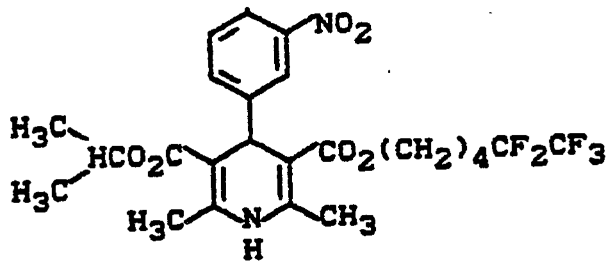

a) Zu einer Lösung von 18 g (50 mmol) racemischen 1,4-Dihydro-2,6-dimethyl-4-(3-nitrophenyl)-pyridin-3,5-dicarbonsäuremonoisopropylesters in 125 ml absolutem Tetrahydrofuran wurden 10 g (62 mmol) 1,1'-Carbonyldiimidazol zugegeben. Die Mischung wurde 45 Minuten bei Raumtemperatur gerührt und anschließend 45 Minuten zum Sieden erhitzt. Nach dem Erkalten wurde das Lösungsmittel im Vakuum abdestilliert, der Rückstand in Dichlormethan aufgenommen und mit verdünnter Natronlauge und Wasser gewaschen. Die organische Phase wurde über wasserfreiem Natriumsulfat getrocknet und unter vermindertem Druck eingeengt. Der ölige Rückstand kristallisierte beim Verreiben mit Ether schnell durch, er wurde abgesaugt und mit Ether gewaschen. Nach dem Trocknen im Vakuum resultierten 19 g (93 % der Theorie) des racemischen 1,4-Dihydro-2,6-dimethyl-4-(3-nitrophenyl)-pyridin-3,5-dicarbonsäure-isopropylester-imidazolids vom Schmelzpunkt 190-192° C.

b) 5,13 g (12,5 mmol) des oben dargestellten racemischen 1,4-Dihydro-2,6-dimethyl-4-(3-nitrophenyl)-pyridin-3,5-dicarbonsäure-isopropylester-imidazolids wurden zusammen mit 4,80 g (25 mmol) 4-Perfluorethyl-butanol-1 in 50 ml absolutem Tetrahydrofuran aufgenommen und nach Zugabe einer Spatelspitze Natriumhydrid 1 Stunde unter Rückfluß erhitzt. Nach dem Erkalten wurde das Lösungsmittel im Vakuum abdestilliert und der Rückstand über Kieselgel mit Dichlormethan/Methanol = 10/1 als Eluierungsmittel gereinigt. Es wurden 4,5 g (67 % der Theorie) des racemischen 1,4-Dihydro-2,6-dimethyl-4-(3-nitrophenyl)-pyridin-3,5-dicarbonsäure-isopropyl-(4-perfluorethyl-butyl)-esters als hochviskoses Öl ($M^+ = 534$) erhalten, N (ber.) 5,24 %, N (gef.) 4,9 %.

Beispiel 4

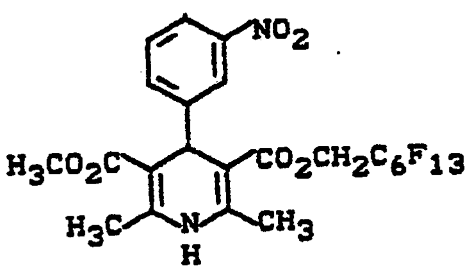

Analog Beispiel 1 wurde durch Umsetzung von 2-(3-Nitro-benzyliden)-acetessigsäuremethylester mit 3-Aminocrotonsäureperfluorhexylmethylester in Ethanol der1,4-Dihydro-2,6-dimethyl-4-(3-nitrophenyl)-pyridin-3,5-dicarbonsäuremethyl-perfluorhexylmethyl-ester vom Fp.: 114° C (Toluol) erhalten. Ausbeute: 67 % der Theorie.

Beispiel 5

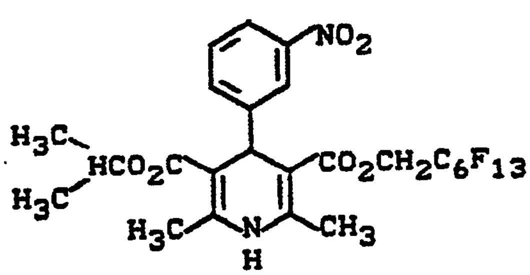

Analog Beispiel 3 wurde durch Umsetzung des 1,4-Dihydro-2,6-dimethyl-4-(3-nitrophenyl)-pyridin-3,5-carbonsäuremonoisopropylesters mit Carbonyldiimidazol und anschließende Alkanolyse des Imidazolids mit Perfluorhexylmethanol in Tetrahydrofuran der1,4-Dihydro-2,6-dimethyl-4-(3-nitrophenyl)-pyridin-3,5-dicarbonsäure-isopropyl-perfluorhexylmethyl-ester vom Fp.: 116° C (Ether/Petrolether) erhalten.
Ausbeute: 63 % der Theorie.

Beispiel 6

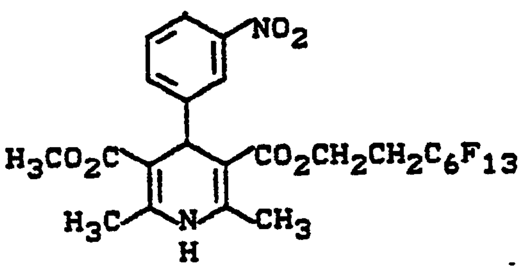

Analog Beispiel 1 wurde durch Umsetzung von 2-(3-Nitrobenzyliden)-acetessigsäuremethylester mit 3-Aminocrotonsäure-(2-perfluorhexyl-ethyl)-ester in Isopropanol der 1,4- Dihydro-2,6-dimethyl-4-(3-nitrophenyl)-pyridin-3,5-dicarbonsäure-methyl-(2-perfluorhexyl-ethyl)-ester vom Fp.: 114° C erhalten.
Ausbeute: 61 % der Theorie.

Beispiel 7

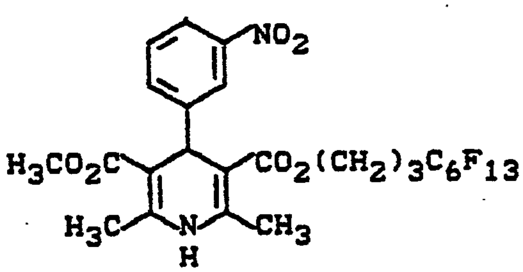

Analog Beispiel 1 wurde durch Umsetzung von 2-(3-Nitrobenzyliden)-acetessigsäuremethylester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-methyl-(3-perfluorhexyl-propyl)-ester vom Fp.: 146° C (Ethanol) erhalten.
Ausbeute: 63 % der Theorie.

Beispiel 8 (Verfahrensvariante G)

(-)-1,4-Dihydro-2,6-dimethyl-4-(3-nitrophenyl)-pyridin-3,5-dicarbonsäure-methyl-(3-perfluorhexyl-propyl)-ester

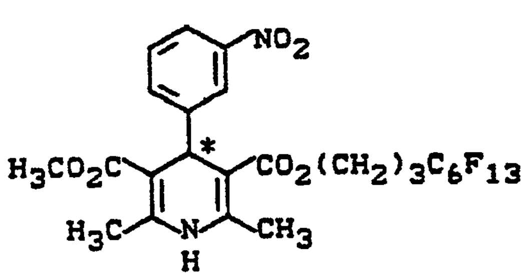

Zu einer Suspension von 21,2 g (63,8 mmol) rechtsdrehendem, enantiomerenreinen, 1,4-Dihydro-2,6-dimethyl-4-(3-nitrophenyl)-pyridin-3,5-dicarbonsäuremonomethylester in 200 ml absolutem Tetrahydrofuran wurden in der Siedehitze portionsweise 12,9 g (79,5 mmol) 1,1'-Carbonyldiimidazol zugegeben. Nach einstündigem Rühren unter Rückfluß wurde die Reaktionsmischung mit 36,15 g (95,7 mmol) 3-Perfluorhexylpropanol-1 und einer Spatelspitze Natriumhydrid versetzt und weitere 2 Stunden zum Sieden erhitzt. Anschließend wurde das Lösungsmittel unter vermindertem Druck abdestilliert und der Rückstand

säulenchromatographisch über Kieselgel mit einem Gemisch von Dichlormethan/Methanol = 10/1 als Elueirungsmittel gereinigt. Das Rohprodukt wurde in der vierfachen Menge Ether/Petrolether = 1/1 ausgerührt, abgesaugt und im Vakuum getrocknet.
Fp.: 133-134° C
Ausbeute: 34,7 g (79 % der Theorie)
$[\alpha]^{20}_D$ = -17,52° (c = 0,626 % w/v, Ethanol).

Beispiel 9 (Verfahrensvariante G)

(+)-1,4-Dihydro-2,6-dimethyl-4-(3-nitrophenyl)-pyridin-3,5-dicarbonsäure-methyl-(3-perfluorhexyl-propyl)-ester

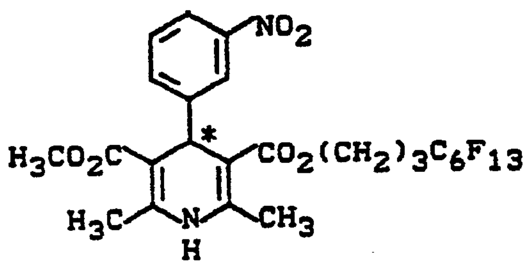

a) In eine Suspension von 30,1 g (90,6 mmol) linksdrehendem, enantiomerenreinen 1,4-Dihydro-2,6-dimethyl-4-(3-nitrophenyl)-pyridin-3,5-dicarbonsäuremonomethylester in 200 ml absolutem Tetrahydrofuran wurden in der Siedehitze unter Rühren portionsweise 18,32 g (113 mmol) 1,1′-Carbonyldiimidazol eingetragen. Die Mischung wurde 1 Stunde unter Rückfluß erhitzt und nach dem Abkühlen mit 400 ml Wasser und 40 ml gesättigter Natriumbicarbonatlösung kräftig durchmischt. Das ausgefallene Produkt wurde abgesaugt, mit Wasser gewaschen und im Vakuum bei 60° C getrocknet. Es resultierten 28,2 g (81 % der Theorie) des rechtsdrehenden, enantiomerenreinen 1,4-Dihydro-2,6-dimethyl-4-(3-nitrophenyl)-pyridin-3,5-dicarbonsäure-methylester-imidazolids vom Fp.: 198° C.
$[\alpha]^{20}_D$ = +166,23° (c = 0,533 % w/v, Ethanol).

b) Eine Mischung von 9,7 g (25,4 mmol) des oben dargestellten rechtsdrehenden 1,4-Dihydro-2,6-dimethyl-4-(3-nitrophenyl)-pyridin-3,5-dicarbonsäure-methylester-imidazolids und 19,03 g (50,8 mmol) 3-Perfluorhexyl-propanol-1 in 100 ml absolutem Tetrahydrofuran wurde nach Zugabe von 2 Spatelspitzen Natriumhydrid 1 Stunde unter Rückfluß erhitzt. Anschließend wurde das Lösungsmittel im Vakuum abdestilliert und der Rückstand über Kieselgel unter Verwendung eines Gemisches von Dichlormethan/Methanol = 1/1 als Eluierungsmittel säulenchromatographisch gereinigt. Das Rohprodukt wurde in der vierfachen Menge Ether/Petrolether = 1/1 ausgerührt, abgesaugt und im Vakuum getrocknet. Es resultierten 14,1 g (80 % der Theorie) des rechtsdrehenden, enantiomerenreinen 1,4-Dihydro-2,6-dimethyl-4-(3-nitrophenyl)-pyridin-3,5-dicarbonsäure-methyl-(3-perfluorhexyl-propyl)-esters vom Fp.: 133-134° C. $[\alpha]^{20}_D$ = + 17,48° (c = 0,976 % w/v, Ethanol).

Beispiel 10

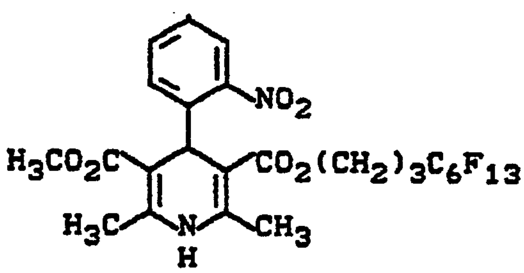

Analog Beispiel 1 wurde durch Umsetzung von 2-(2-Nitrobenzyliden)-acetessigsäuremethylester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-methyl-(3-perfluorhexyl-propyl)-ester vom Fp.: 98° C (Ether/Petrolether) erhalten.
Ausbeute: 53 % der Theorie.

Beispiel 11

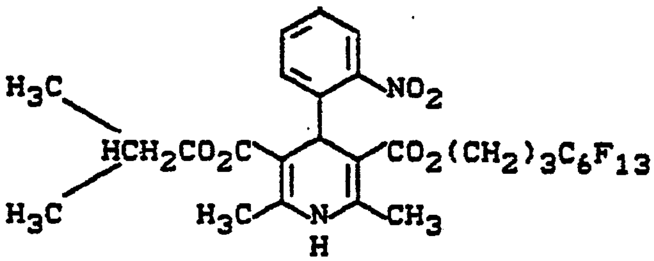

Analog Beispiel 1 wurde durch Umsetzung von 2-(2-Nitro-benzliden)-acetessigsäureisobutylester mit 3-Aminocrotonsäure-(3-perfluorhexyl-propyl)-ester in Ethanol der 1,4-Dihydro-2,6-dimethyl-4-(2-nitrophenyl)-pyridin-3,5-dicarbonsäure-isobutyl-(3-perfluorhexyl-propyl)-ester als hochviskoses Öl ($M^+$ = 734) erhalten.
Ausbeute: 49 % der Theorie
C (ber.) 44.9 % C (gef.) 45.1 %.

Beispiel 12

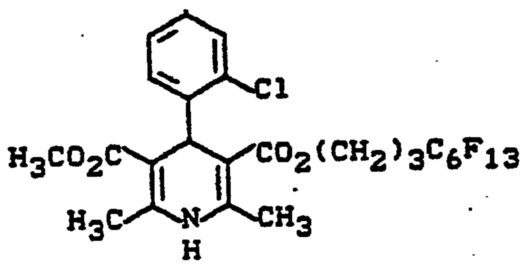

Analog Beispiel 1 wurde durch Umsetzung von 2-(2-Chlorbenzyliden)-acetessigsäuremethylester mit 3-Aminocrotonsäure-(3-perfluorhexyl-propyl)-ester in Methanol der 1,4-Dihydro-2,6-dimethyl-4-(2-chlorphenyl)-pyridin-3,5-dicarbonsäure-methyl-(3-perfluorhexyl-propyl)-ester vom Fp.: 107° C (Toluol) erhalten.
Ausbeute: 52 % der Theorie.

Beispiel 13

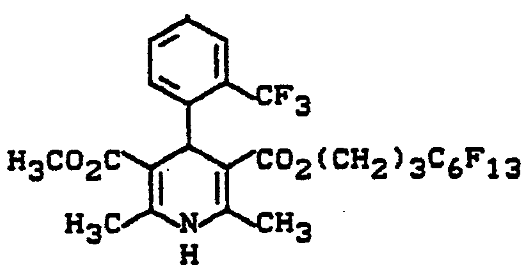

Analog Beispiel 1 wurde durch Umsetzung von 2-(2-Trifluormethylbenzyliden)-acetessigsäuremethylester mit 3-Aminocrotonsäure-(3-perfluorhexyl-propyl)-ester in Isopropanol der1,4-Dihydro-2,6-dimethyl-4-(2-trifluormethylphenyl)-pyridin-3,5-dicarbonsäure-methyl-(3-perfluorhexyl-propyl)-ester vom Fp.: 110° C (Ether/Petrolether) erhalten.
Ausbeute: 46 % der Theorie.

Beispiel 14

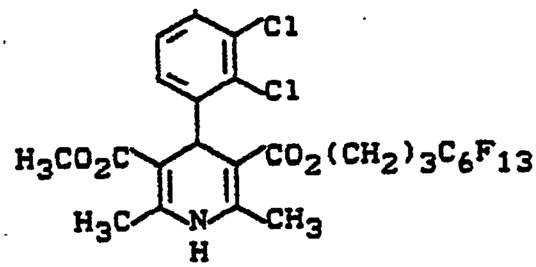

Analog Beispiel 1 wurde durch Umsetzung von 2-(2,3-Dichlorbenzyliden)-acetesssigsäuremethylester mit 3-Aminocrotonsäure-(3-perfluorhexyl-propyl)-ester in Ethanol der 1,4-Dihydro-2,6-dimethyl-4-(2,3-dichlorphenyl)-pyridin-3,5-dicarbonsäure-methyl-(3-perfluorhexyl-propyl)-ester vom Fp.: 100° C (Petrolether) erhalten.
Ausbeute: 50 % der Theorie.

Beispiel 15

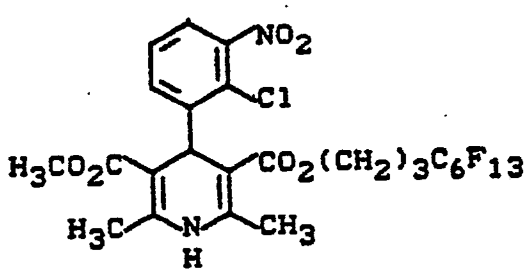

Analog Beispiel 1 wurde durch Umsetzung von 2-(2-Chlor-3-nitrobenzyliden)-acetessigsäuremethylester mit 3-Aminocrotonsäure-(3-perfluorhexyl-propyl)-ester in Ethanol der 1,4-Dihyro-2,6-dimethyl-4-(2-chlor-3-nitrophenyl)-pyridin-3,5-dicarbonsäure-methyl-(3-perfluorhexyl-propyl)-ester als gelbes Öl ($M^+ = 726$) erhalten.
Ausbeute: 53 % der Theorie.

Beispiel 16

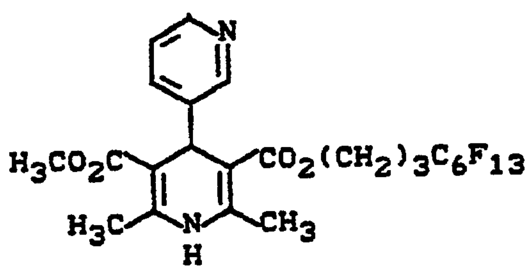

Analog Beispiel 1 wurde durch Umsetzung von 2-Acetyl-3-(pyridyl-3-)-acrylsäuremethylester mit 3-Aminocrotonsäure(3-perfluorhexyl-propyl)-ester in Ethanol der 1,4-Dihydro-2,6-dimethyl-4-(pyridyl-3)-pyridin-3,5-dicarbonsäuremethyl-(3-perfluorhexyl-propyl)-ester vom Fp.: 155° C (Ether) erhalten.
Ausbeute: 57 % der Theorie.

Beispiel 17

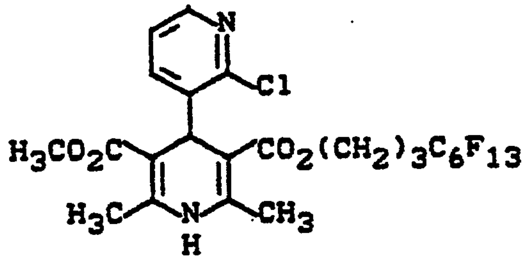

Analog Beispiel 1 wurde durch Umsetzung von 2-Acetyl-3-(2-chlor-pyridyl-3-)-acrylsäuremethylester mit 3-Aminocrotonsäure-(3-perfluorhexyl-propyl)-ester in Isopropanol der 1,4-Dihydro-2,6-dimethyl-4-(2-chlor-pyridyl-3)-pyridin-3,5-dicarbonsäure-methyl-(3-perfluorhexyl-propyl)-ester vom Fp.: 155° C erhalten.
Ausbeute: 48 % der Theorie.

Beispiel 18

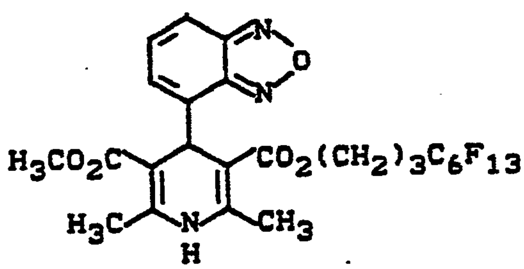

Analog Beispiel 1 wurde durch Umsetzung von 2-Acetyl-3-(2,1,3-benzoxadiazolyl-4)-acrylsäuremethylester mit 3-Aminocrotonsäure-(3-perfluorhexyl-propyl)-ester in Propanol der 1,4-Dihydro-2,6-dimethyl-4-(2,1,3-benzoxadiazolyl-4)-pyridin-3,5-dicarbonsäure-methyl-(3-perfluorhexyl-propyl)-ester vom Fp.: 110° C (Ether) erhalten.
Ausbeute: 43 % der Theorie.

Beispiel 19

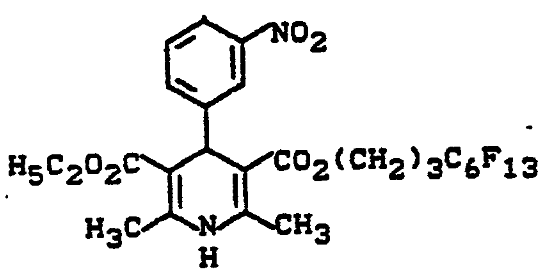

Analog Beispiel 1 wurde durch Umsetzung von 2-(3-Nitrobenzyliden)-acetessigsäureethylester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-ethyl-(3-perfluorhexyl-propyl)-ester vom Fp.: 119° C (Ethanol) erhalten.
Ausbeute: 59 % der Theorie.

Beispiel 20

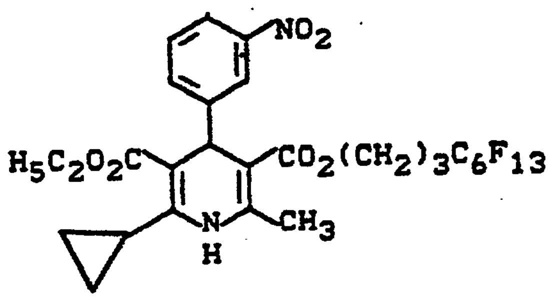

Analog Beispiel 1 wurde durch Umsetzung von 2-Cyclopropylcarbonyl-3-(3-nitrophenyl)-acrylsäureethylester mit 3-Aminocrotonsäure-(3-perfluorhexyl-propyl)-ester in Isopropanol der 1,4-Dihydro-2-cyclopropyl-6-methyl-4-(3-nitrophenyl)-pyridin-3,5-dicarbonsäure-3-ethyl-5-(3-perfluorhexyl-propyl)-ester vom Fp.: 112° C (Ether/Petrolether) erhalten.
Ausbeute: 42 % der Theorie.

Beispiel 21

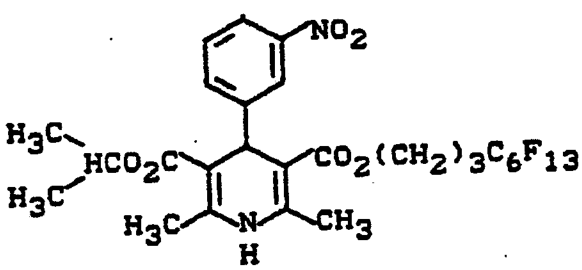

Analog Beispiel 1 wurde durch Umsetzung von 2-(3-Nitrobenzyliden)-acetessigsäureisopropylester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-isopropanol-(3-perfluorhexyl-propyl)-ester vom Fp.: 109° C (Ethanol) erhalten.
Ausbeute: 65 % der Theorie.

Beispiel 22

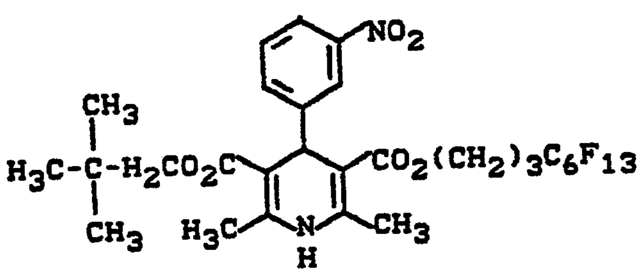

Analog Beispiel 1 wurde durch Umsetzung von 2-(3-Nitrobenzyliden)-acetessigsäureneopentylester mit 3-Aminocrotonsäure-(3-perfluorhexyl-propyl)-ester in Isopropanol der1,4-Dihydro-2,6-dimethyl-4-(3-nitrophenyl)-pyridin-3,5-dicarbonsäure-neopentyl-(3-perfluorhexyl-propyl)-ester vom Fp.: 130° C (Ether/Petrolether) erhalten.
Ausbeute: 68 % der Theorie.

Beispiel 23

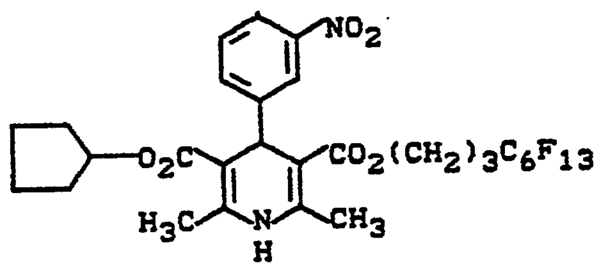

Analog Beispiel 1 wurde durch Umsetzung von 2-(3-Nitrobenzyliden)-acetessigsäurecyclopentylester mit 3-Aminocrotonsäure-(3-perfluorhexyl-propyl)-ester in Ethanol der 1,4-Dihydro-2,6-dimethyl-4-(3-nitrophenyl)-pyridin-3,5-dicarbonsäure-cyclopentyl-(3-perfluorhexyl-propyl)-ester vom Fp.: 118° C (Ether/Petrolether) erhalten.
Ausbeute: 68 % der Theorie.

Beispiel 24

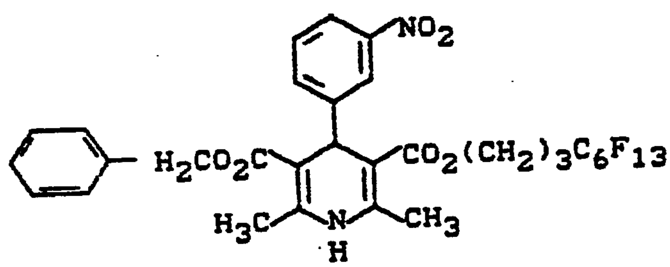

Analog Beispiel 1 wurde durch Umsetzung von 2-(3-Nitrobenzyliden)-acetessigsäurebenzylester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-benzyl-(3-perfluorhexyl-propyl)-ester vom Fp.: 109° C (Ethanol ) erhalten.
Ausbeute: 71 % der Theorie.

Beispiel 25

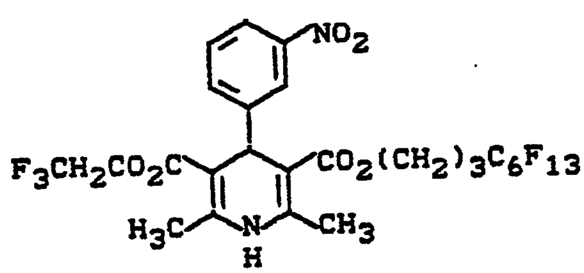

Analog Beispiel 8 wurde durch Umsetzung des racemischen 1,4-Dihydro-2,6-dimethyl-4-(3-nitrophenyl)-pyridin-3,5-dicarbonsäuremono-(3-perfluorhexyl-propyl)-esters mit 1,1'-Carbonyldiimidazol in Tetrahydrofuran und anschließende Alkanolyse des entstandenen 1,4-Dihydro- 2,6-dimethyl-4-(3-nitrophenyl)-pyridin-3,5-dicarbonsäure-(3-perfluorhexyl-propyl)-ester-imidazolids mit 2,2,2-Trifluorethanol der 1,4-Dihydro-2,6-dimethyl-4-(3-nitrophenyl)-pyridin-3,5-dicarbonsäure-(2,2,2-trifluorethyl)-(3-perfluorhexyl-propyl)-ester vom Fp.: 137° C (Petrolether) erhalten.
Ausbeute: 81 % der Theorie.

Beispiel 26

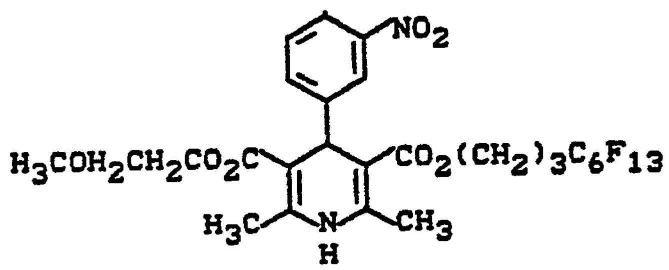

Analog Beispiel 1 wurde durch Umsetzung von 2-(3-Nitrobenzyliden)-acetessigsäure-(2-methoxyethyl)-ester mit 3-Aminocrotonsäure-(3-perfluorhexyl-propyl)-ester in Isopropanol der 1,4-Dihydro-2,6-dimethyl-4-(3-nitrophenyl)-pyridin-3,5-dicarbonsäure-(2-methoxyethyl)-(3-perfluorhexyl-propyl)-ester vom Fp.: 127° C (Ether/Petrolether) erhalten.
Ausbeute: 75 % der Theorie.

Beispiel 27

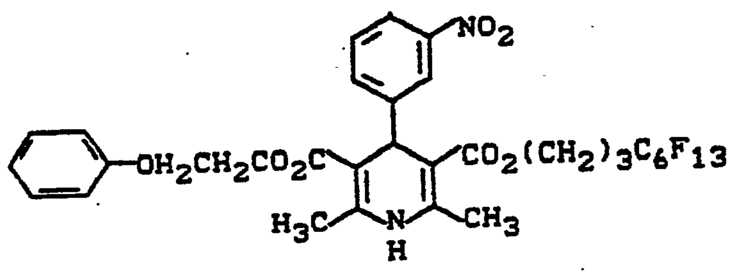

Analog Beispiel 8 wurde durch Umsetzung des racemischen 1,4-Dihydro-2,6-dimethyl-4-(3-nitrophenyl)-pyridin-3,5-dicarbonsäuremono-(3-perfluorhexyl-propyl)-esters mit 1,1′-Carbonyldiimidazol in Tetrahydrofuran und anschließende Alkanolyse des entstandenen 1,4-Dihydro-2,6-dimethyl-4-(3-nitrophenyl)-pyridin-3,5-dicarbonsäure-(3-perfluorhexyl-propyl)-ester-imidazolids mit 2-Phenoxyethanol der 1,4-Dihydro-2,6-dimethyl-4-(3-nitrophenyl)-pyridin-3,5-dicarbonsäure-(2-phenoxyethyl)-(3-perfluorhexyl-propyl)-ester vom Fp.: 113° C (Ether/Petrolether) erhalten.
Ausbeute: 51 % der Theorie.

Beispiel 28

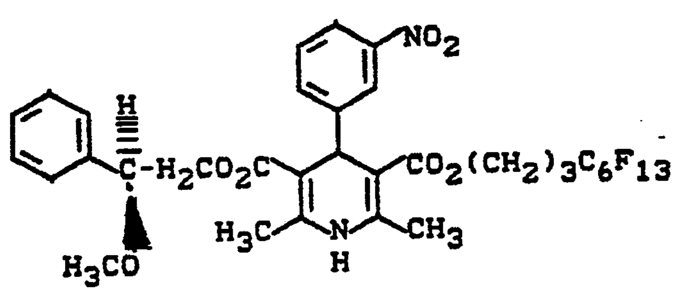

Analog Beispiel 2 wurde durch Umsetzung von 2-(3-Nitrobenzyliden)-acetessigsäure-(3-perfluorhexyl-propyl)-ester mit 3-Aminocrotonsäure-[(S)-2-methoxy-2-phenylethyl]-ester in Ethanol der 1,4-Dihydro-2,6-dimethyl-4-(3-nitrophenyl)-pyridin-3,5-dicarbonsäure-[(S)-2-methoxy-2-phenylethyl]-(3-perfluorhexyl-propyl)-ester vom Fp.: 79° C (Toluol) erhalten.
Ausbeute: 41 % der Theorie.

Beispiel 29

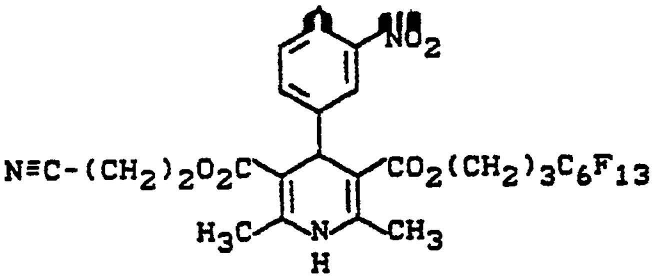

Analog Beispiel 1 wurde durch Umsetzung von 2-(3-Nitrobenzyliden)-acetessigsäure-(2-cyanoethyl)-ester mit 3-Aminocrotonsäure-(3-perfluorhexyl-propyl)-ester in Ethanol der 1,4-Dihydro-2,6-dimethyl-4-(3-nitrophenyl)-pyridin-3,5-dicarbonsäure-(2-cyanoethyl)-(3-perfluorhexyl-propyl)-ester vom Fp.: 138° C (Ethanol) erhalten.
Ausbeute: 75 % der Theorie.

Beispiel 30 (Verfahrensvariante G)

1,4-Dihydro-2,6-dimethyl-4-(3-nitrophenyl)-pyridin-3,5-dicarbonsäure-(2-dimethylaminoethyl)-(3-perfluorhexyl-propyl)-ester

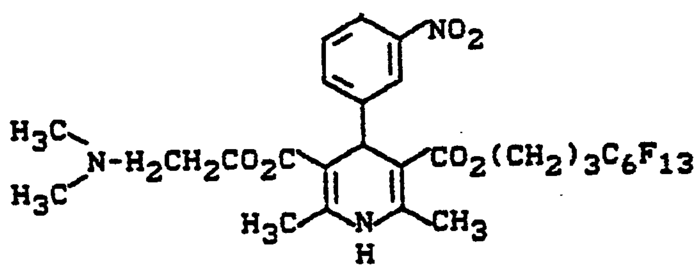

a) 10,2 g (15 mmol) 1,4-Dihydro-2,6-dimethyl-4-(3-nitrophenyl)-pyridin-3,5-dicarbonsäuremono-(3-perfluorhexyl-propyl)-ester wurden in 30 ml absolutem Tetrahydrofuran aufgenommen und nach Zugabe von 3,65 g (22,5 mmol) 1,1'-Carbonyldiimidazol 1 Stunde unter Rückfluß erhitzt. Nach dem Erkalten wurde die Reaktionslösung mit 100 ml Dichlormethan und 50 ml verdünnter Natronlauge kräftig durchmischt, die organische Phase anschließend abgetrennt, mit Wasser gewaschen und nach dem Trocknen über Natriumsulfat unter vermindertem Druck eingeengt. Der Rückstand wurde in einem Gemisch von gleichen Teilen Ether und Petrolether verrührt, abgesaugt und im Vakuum getrocknet. Es resultierten 7,7 g (71 %) des 1,4-Dihydro-2,6-dimethyl-4-(3-nitrophenyl)-pyridin-3,5-dicarbonsäure-(3-perfluorhexylpropyl)-ester-imidazolids vom Fp.: 156° C.

b) 7,28 g (10 mmol) des oben dargestellten 1,4-Dihydro-2,6-dimethyl-4-(3-nitrophenyl)-pyridin-3,5-dicarbonsäure-(3-perfluorhexyl-propyl)-ester-imidazolids wurden zusammen mit 1,34 g (15 mmol) 2-Dimethylaminoethanol in 50 ml absolutem Tetrahydrofuran aufgenommen und nach Zugabe einer Spatelspitze Natriumhydrid 1 Stunde bei Raumtemperatur gerührt. Anschließend wurde das Lösungsmittel unter vermindertem Druck abdestilliert und der Rückstand säulenchromatographisch über Kieselgel mit einem Gemisch aus Dichlormethan/Methanol = 10/1 als Eluierungsmittel gereinigt. Das Produkt wurde in Ether/Petrolether=1/1 verrührt und abgesaugt. Nach dem Trocknen hinterblieben 4,9 g (65 %) des 1,4-Dihydro-2,6-dimethyl-4-(3-nitrophenyl)-pyridin-3,5-dicarbonsäure-(2-dimethylaminoethyl)-(3-perfluorhexyl-propyl)-esters vom Fp.: 98° C.

Beispiel 31

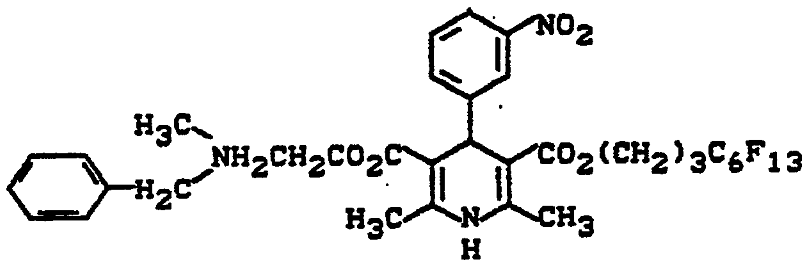

Analog Beispiel 30 wurde durch Alkanoylse des 1,4-Dihydro-2,6-dimethyl-4-(3-nitrophenyl)-pyridin-3,5-dicarbonsäure-(3-perfluorhexyl-propyl)-ester-imidazolids mit 2-(N- Benzyl-N-methyl-amino)-ethanol in Tetrahydrofuran der 1,4-Dihydro-2,6-dimethyl-4-(3-nitrophenyl)-piperidin-3,5-dicarbonsäure-(2-N-benzyl-N-methylamino-ethyl)-(3-perfluorhexyl-propyl)-ester vom Fp.: 96° C erhalten.
Ausbeute: 70 % der Theorie.

Beispiel 32

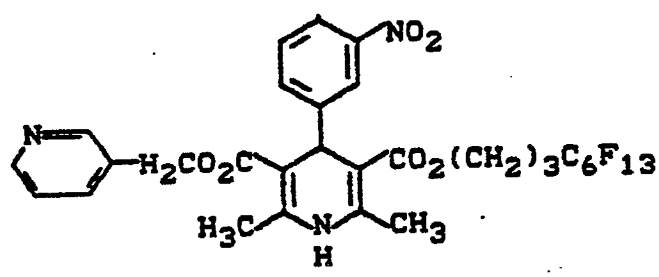

Analog Beispiel 30 wurde durch Alkanolyse des 1,4-Dihydro-2,6-dimethyl-4-(3-nitrophenyl)-pyridin-3,5-dicarbonsäure-(3-perfluorhexyl-propyl)-ester-imidazolids mit 3-Hydroxymethylpyridin in Tetrahydrofuran der 1,4-Dihydro-2,6-dimethyl-4-(3-nitrophenyl)-pyridin-3,5-dicarbonsäure-(3-pyridylmethyl)-(3-perfluorhexyl-propyl)-ester vom Fp.: 155° C erhalten.
Ausbeute: 55 % der Theorie.

Beispiel 33

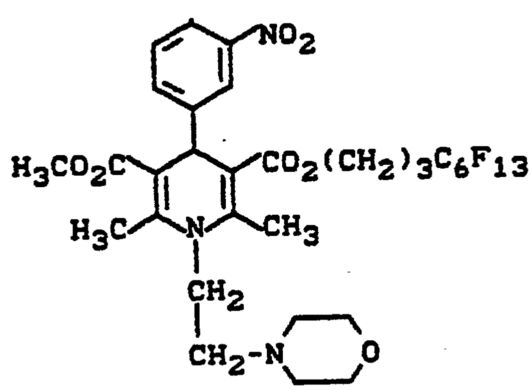

Analog Beispiel 1 wurde durch Umsetzung des 2-(3-Nitrobenzyliden)-acetessigsäuremethylesters mit 3-(2-N-Morpholinoethylamino)-crotonsäure-(3-perfluorhexyl-propyl)-ester in Pyridin/Eisessig der 1,4-Dihydro-2,6-dimethyl-1-(2-N-morpholinoethyl)-4-(3-nitrophenyl)-pyridin-3,5-dicarbonsäure-methyl-(3-perfluorhexyl-propyl)-ester vom Fp.: 70° C (Ether) erhalten.
Ausbeute: 39 % der Theorie.

Beispiel 34

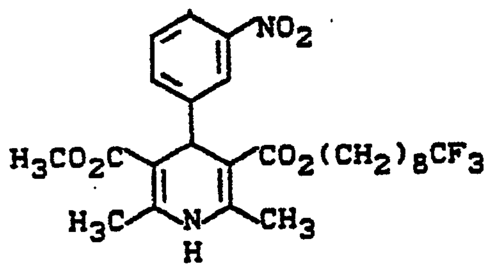

Analog Beispiel 1 wurde durch Umsetzung von 2-(3-Nitrobenzyliden)-acetessigsäuremethylester mit 3-Aminocrotonsäure-(9,9,9-trifluornonyl)-ester in Isopropanol der 1,4-Dihydro-2,6-dimethyl-4-(3-nitrophenyl)-pyridin-3,5-dicarbonsäure-methyl-(9,9,9-trifluornonyl)-ester vom Fp.: 136° C (Ethanol) erhalten.
Ausbeute: 70 % der Theorie.

Beispiel 35

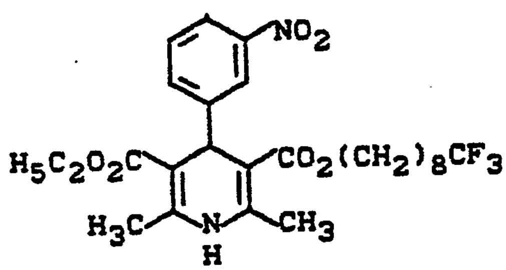

Analog Beispiel 1 wurde durch Umsetzung von 2-(3-Nitrobenzyliden)-acetessigsäureethylester mit 3-Aminocrotonsäure-(9,9,9-trifluornonyl)-ester in Ethanol der 1,4-Dihydro-2,6-dimethyl-4-(3-nitrophenyl)-pyridin-3,5-dicarbonsäure-ethyl-(9,9,9-trifluornonyl)-ester vom Fp.: 110° C (Ethanol) erhalten.
Ausbeute: 66 % der Theorie.

Beispiel 36

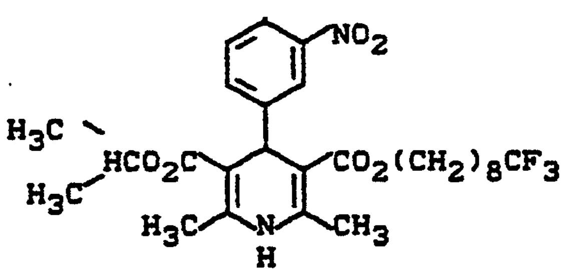

Analog Beispiel 1 wurde durch Umsetzung von 2-(3-Nitrobenzyliden)-acetessigsäureisopropylester mit 3-Aminocrotonsäure-(9,9,9-trifluornonyl)-ester in Isopropanol der 1,4-Dihydro-2,6-dimethyl-4-(3-nitrophenyl)-pyridin-3,5-dicarbonsäure-isopropyl-(9,9;9-trifluornonyl)-ester vom Fp.: 114° C (Ethanol) erhalten.
Ausbeute: 61 % der Theorie.

Beispiel 37

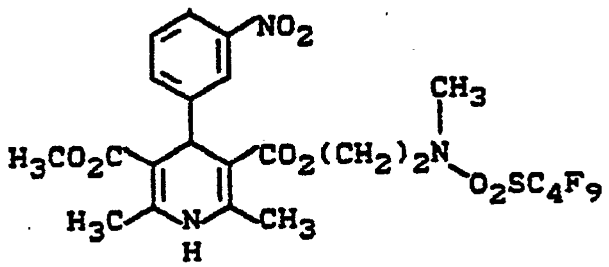

Analog Beispiel 1 wurde durch Umsetzung von 2-(3-Nitrobenzyliden)-acetessigsäuremethylester mit 3-Aminocrotonsäure-[2-(N-methyl-N-perfluorbutylsulfonylamino)-ethyl]- ester in Isopropanol der 1,4-Dihydro-2,6-dimethyl-4-(3-nitrophenyl)-pyridin-3,5-dicarbonsäure-methyl-[2-(N-methyl-N-perfluorbutylsulfonylamino)-ethyl]-ester vom Fp.: 115° C (Isopropanol) erhalten.
Ausbeute: 39 % der Theorie.

Beispiel 38

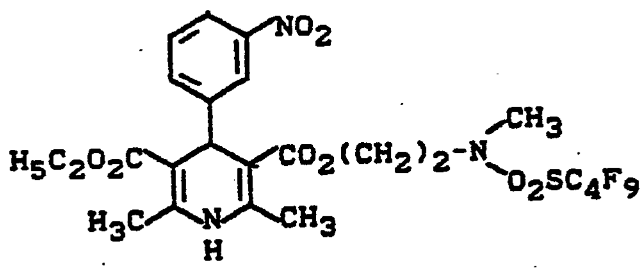

Analog Beispiel 1 wurde durch Umsetzung von 2-(3-Nitrobenzyliden)-acetessigsäureethylester mit 3-Aminocrotonsäure-[2-(N-methyl-N-perfluorbutylsulfonylamino)-ethyl]-ester in Isopropanol der 1,4-Dihydro-2,6-dimethyl-4-(3-nitrophenyl)-pyridin-3,5-dicarbonsäure-ethyl-[2-(N-methyl-N-perfluorbutylsulfonylamino)-ethyl]-ester vom Fp.: 55° C (Isopropanol) erhalten.
Ausbeute: 55 % der Theorie.

Beispiel 39

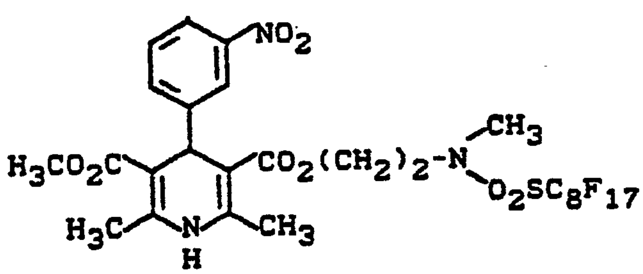

Analog Beispiel 1 wurde durch Umsetzung von 2-(3-Nitrobenzyliden)-acetessigsäuremethylester mit 3-Aminocrotonsäure-[2-(N-methyl-N-perfluoroctylsulfonylamino)-ethyl]-ester in Methanol der 1,4-Dihyro-2,6-dimethyl-4-(3-nitrophenyl)-pyridin-3,5-dicarbonsäure-methyl-[2-(N-methyl-N-perfluoroctylsulfonylamino)-ethyl]-ester vom Fp.: 136° C (Essigester) erhalten.
Ausbeute: 33 % der Theorie.

Beispiel 40

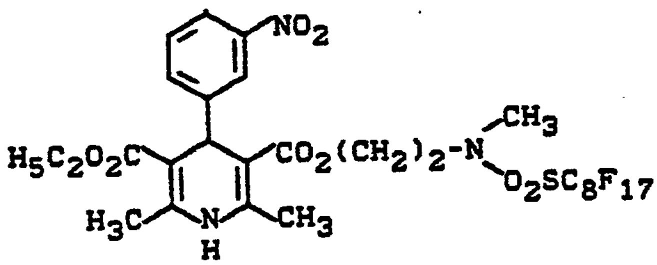

Analog Beispiel 1 wurde durch Umsetzung von 2-(3-Nitrobenzyliden)-acetessigsäureethylester mit 3-Aminocroton säure-[2-(N-methyl-N-perfluoroctylsulfonylamino)-ethyl]-ester in Ethanol der 1,4-Dihydro-2,6-dimethyl-4-(3-nitrophenyl)-pyridin-3,5-dicarbonsuäre-ethyl-[2-(N-methyl-N-perfluoroctylsulfonylamino)-ethyl]-ester vom Fp.: 124° C (Isopropanol) erhalten.
Ausbeute: 35 %.

Beispiel 41

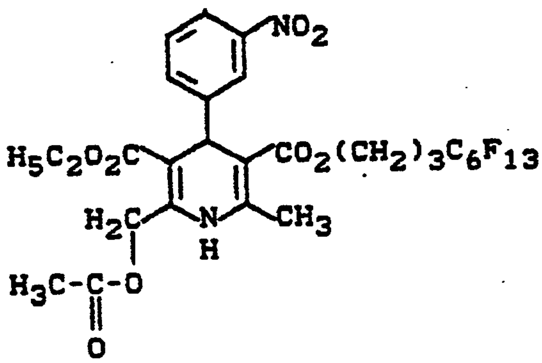

Analog Beispiel 1 wurde durch Umsetzung von 4-Acetoxy-2-(3-nitrobenzyliden)-acetessigsäureethylester mit 3-Aminocrotonsäure-(3-perfluorhexyl-propyl)-ester in Isopropanol der 1,4-Dihydro-2-acetoxymethyl-6-methyl-4-(3-nitrophenyl)-pyridin-3,5-dicarbonsäure-3-ethyl-5-(3-perfluorhexyl-propyl)-ester vom Fp.: 78° C (Ether/Petrolether) erhalten.
Ausbeute: 59 % der Theorie.

Beispiel 42

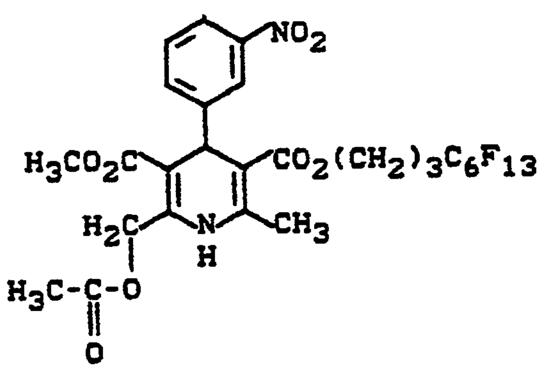

Analog Beispiel 1 wurde durch Umsetzung von 4-Acetoxy-2-(3-nitrobenzyliden)-acetessigsäuremethylester mit 3-Aminocrotonsäure-(3-perfluorhexyl-propyl)-ester in Isopropanol der 1,4-Dihydro-2-acetoxymethyl-6-methyl-4-(3-nitrophenyl)-pyridin-3,5-dicarbonsäure-3-methyl-5-(3-perfluorhexyl-propyl)-ester vom Fp.: 72° C (Ether/Petrolether) erhalten.
Ausbeute: 63 % der Theorie.

Beispiel 43

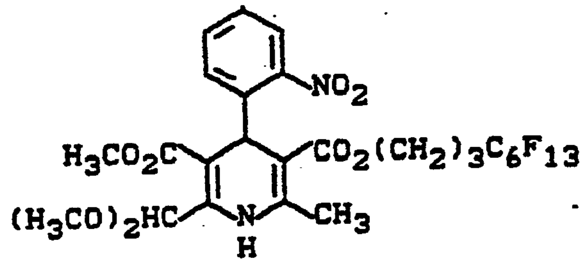

Analog Beispiel 1 wurde durch Umsetzung von 4,4-Dimethoxy-2-(2-nitrobenzyliden)-acetessigsäuremethylester mit 3- Aminocrotonsäure-(3-perfluorhexyl-propyl)-ester in Methanol der 1,4-Dihydro-2-dimethoxymethyl-6-methyl-4-(2-nitrophenyl)-pyridin-3,5-dicarbonsäure-3-methyl-5-(3-perfluorhexyl-propyl)-ester als hochviskoses Öl ($M^+ = 752$) erhalten.
Ausbeute: 53 % der Theorie.

Beispiel 44

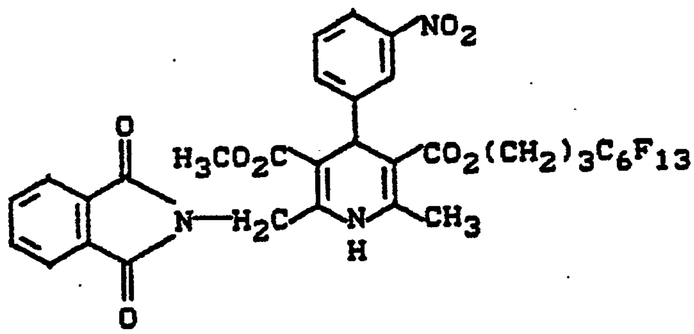

Analog Beispiel 1 wurde der 4-Phthalimido-2-(3-nitrobenzyliden)-acetessigsäuremethylester mit 3-Aminocrotonsäure-(3-perfluorhexyl-propyl)-ester in Methanol zur Reaktion gebracht. Anschließend wurde das Lösungsmittel im Vakuum abdestilliert und der Rückstand in Toluol in Gegenwart katalytischer Mengen p-Toluolsulfonsäure am Wasserabscheider erhitzt. Die Aufarbeitung lieferte den 1,4-Dihydro-2-phthalimidomethyl-6-methyl-4-(3-nitrophenyl)-pyridin-3,5-dicarbonsäure-3-methyl-5-(3-perfluorhexyl-propyl)-ester vom Fp.: 132° C (Ether).
Ausbeute: 48 % der Theorie.

Beispiel 45

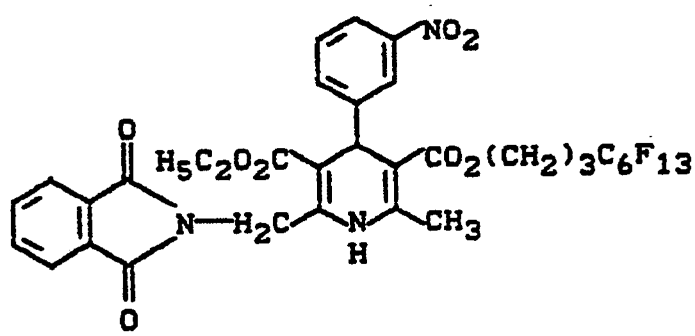

Analog Beispiel 44 wurde aus 4-Phthalimido-2-(3-nitrobenzyliden)-acetessigsäureethylester und 3-Aminocrotonsäure-(3-perfluorhexyl-propyl)-ester der 1,4-Dihydro-2-phthalimidomethyl-6-methyl-4-(3-nitrophenyl)-pyridin-3,5-dicarbonsäure-3-ethyl-5-(3-perfluorhexyl-propyl)-ester vom Fp.: 98° C (Ether) hergestellt.
Ausbeute: 52 % der Theorie.

Beispiel 46

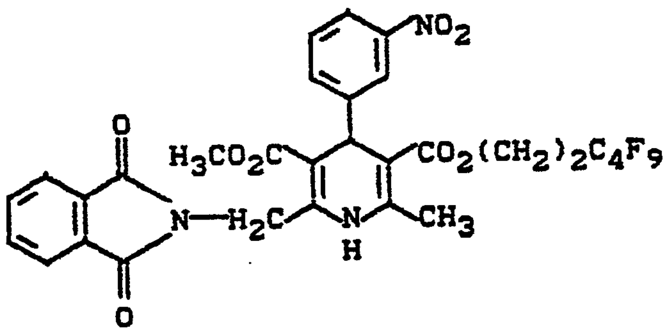

Analog Beispiel 44 wurde aus 4-Phthalimido-2-(3-nitrobenzyliden)-acetessigsäuremethylester und 3-Aminocrotonsäure-(2-perfluorbutyl-ethyl)-ester der 1,4-Dihydro-2-phthalimidomethyl-6-methyl-4-(3-nitrophenyl)-pyridin-3,5-dicarbonsäure-methyl-(2-perfluorbutyl-ethyl)-ester vom Fp.: 111° C (Ether) hergestellt.
Ausbeute: 45 % der Theorie.

Beispiel 47

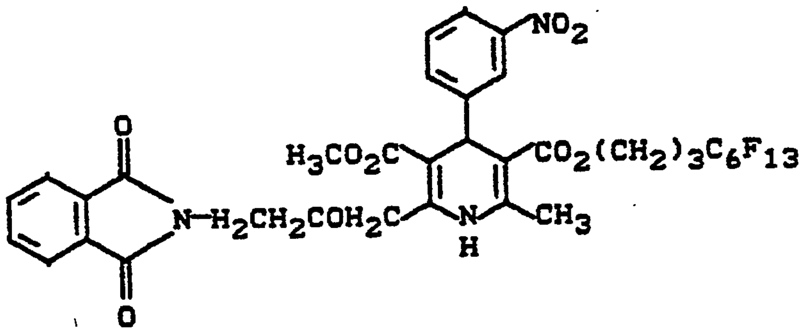

Analog Beispiel 1 wurde durch Umsetzung des 4-(2-Phthalimidoethoxy)-2-(3-nitrobenzyliden)-acetessigsäuremethylesters mit 3-Aminocrotonsäure-(3-perfluorhexyl-propyl)-ester in Isopropanol der 1,4-Dihydro-2-(2-phthalimidoethoxymethyl)-6-methyl-4-(3-nitrophenyl)-pyridin-3,5-dicarbonsäure-3-methyl-5-(3-perfluorhexyl-propyl)-ester vom Fp.: 99° C (Ether/Petrolether) erhalten.
Ausbeute: 42 % der Theorie.

Beispiel 48 (Verfahrensvariante H)

1,4-Dihydro-2-(2-aminoethoxymethyl)-6-methyl-4-(3-nitrophenyl)-pyridin-3,5-dicarbonsäure-3-methyl-5-(3-perfluorhexyl-propyl)-ester

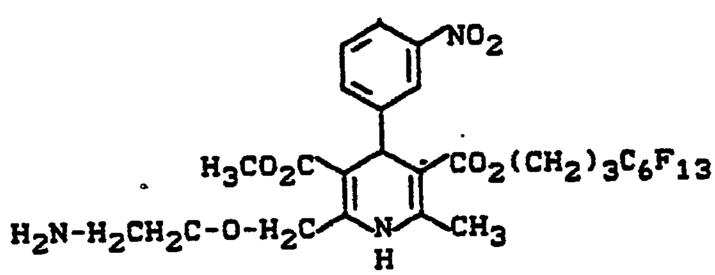

4,9 g (5,6 mmol) 1,4-Dihydro-2-(2-phthalimidoethoxymethyl)-6-methyl-4-(3-nitrophenyl)-pyridin-3,5-dicarbonsäure-3-methyl-5-(3-perfluorhexyl-propyl)-ester (Beispiel 47) wurden zusammen mit 1,4 g (27 mmol) Hydrazinhydrat in 75 ml Ethanol 30 Minuten unter Rückfluß erhitzt. Anschließend wurde vom Ungelösten abfiltriert, das Filtrat mit 300 ml Wasser versetzt und zweimal mit je 100 ml Dichlormethan extrahiert. Die organischen Phasen wurden nach dem Trocknen über Natriumsulfat im Vakuum eingeengt, der feste Rückstand säulenchromatographisch über Kieselgel mit Methanol als Eluierungsmittel gereinigt und über Diphosphorpentoxid getrocknet.
Fp.: 77-78° C
Ausbeute: 1,8 g (44 % der Theorie).

Beispiel 49

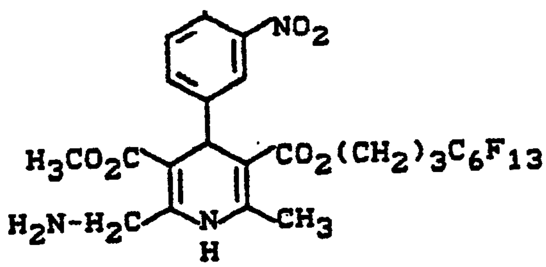

Analog Beispiel 48 wurde durch Umsetzung des 1,4-Dihydro-2-phthalimidomethyl-6-methyl-4-(3-nitrophenyl)-pyridin-3,5-dicarbonsäure-3-methyl-5-(3-perfluorhexyl-propyl)-esters (Beispiel 44) mit Hydrazin in Ethanol der 1,4-Dihydro-2-aminomethyl-6-methyl-4-(3-nitrophenyl)-pyridin-3,5-dicarbonsäure-3-methyl-5-(3-perfluorhexyl-propyl)-ester als gelbes Öl erhalten.
Ausbeute: 39 % der Theorie.
N (ber.) 5,9 % N (gef.) 5,8 %.

Beispiel 50 (Verfahrensvariante H)

1,4-Dihydro-2-cyano-6-methyl-4-(2-nitrophenyl)-pyridin-3,5-dicarbonsäure-3-methyl-5-(3-perfluorhexyl-propyl)-ester

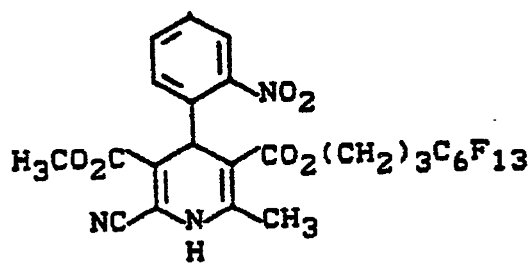

4 g (5,3 mmol) 1,4-Dihydro-2-dimethoxymethyl-6-methyl-4-(2-nitrophenyl)-pyridin-3,5-dicarbonsäure-3-methyl-5-(3-perfluorhexyl-propyl)-ester (Beispiel 43) wurden in 100 ml Aceton aufgenommen und nach Zugabe von 10 ml 20 %iger Salzsäure 2 Stunden bei Raumtemperatur gerührt. Anschließend wurde die Reaktionsmischung im Vakuum eingeengt, der Rückstand in Wasser aufgenommen, die wäßrige Phase mit einer Natriumbicarbonatlösung neutralisiert und mit Dichlormethan extrahiert. Die organischen Extrakte wurden nach Trocknen über wasserfreiem Natriumsulfat unter vermindertem Druck eingeengt, es resultierten 2 g (54 %) des 1,4-Dihydro-2-formyl-6-methyl-4-(2-nitrophenyl)-pyridin-3,5-dicarbonsäure-3-methyl-5-(3-perfluorhexyl-propyl)-esters als gelbes Öl, das in 100 ml Eisessig aufgenommen und nach Zugabe von 0,22 g Hydroxylamin-Hydrochlorid und 0,42 g Natriumacetat 3 Stunden bei Raumtemperatur gerührt wurde. Anschließend wurden 24 ml Essigsäureanhydrid und 5,5 g Natriumacetat zugegeben und die Reaktionsmischung 24 Stunden unter Rückfluß erhitzt. Nach dem Erkalten wurde das Lösungsmittel unter Vakuum abdestilliert, der Rückstand mit Natriumbicarbonat neutralisiert und die wäßrige Phase mit Essigester extrahiert. Die organischen Extrakte wurden über wasserfreiem Natriumsulfat getrocknet, unter vermindertem Druck eingeengt und der Rückstand über Kieselgel mit Dichlormethan/Essigester = 4/1 säulenchromatographisch gereinigt, 0,9 g (44 %) eines gelben Öls ($M^+ = 703$).

Beispiel 51 (Verfahrensvariante H)

1,4-Dihydro-2-hydroxymethyl-6-methyl-4-(3-nitrophenyl)-pyridin-3,5-dicarbonsäure-3-ethyl-5-(3-perfluorhexyl-propyl)-ester

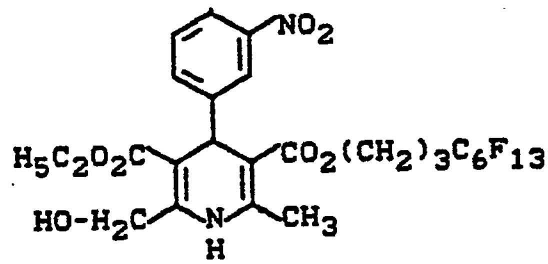

2 g (2,6 mmol) 1,4-Dihydro-2-acetoxymethyl-6-methyl-4-(3-nitrophenyl)-pyridin-3,5-dicarbonsäure-3-ethyl-5-(3-perfluorhexyl-propyl)-ester (Beispiel 41) wurden in 30 ml Methanol gelöst und nach Zugabe katalytischer Mengen Natriummethylat 15 Minuten bei Raumtemperatur gerührt. Anschließend wurde mit Eisessig neutralisiert und die Reaktionmischung im Vakuum eingeengt. Der Rückstand wurde in Dichlormethan aufgenommen, die organische Phase mit Wasser gewaschen und über Natriumsulfat getrocknet. Nach Verdampfen des Lösunbgsmittels wurde das ölige Rohprodukt über Kieselgel mit Dichlormethan/Ethanol = 20/1 säulenchromatographisch gereinigt, Fp.: 124-125° C.
Ausbeute 1 g (53 % der Theorie).

Beispiel 52

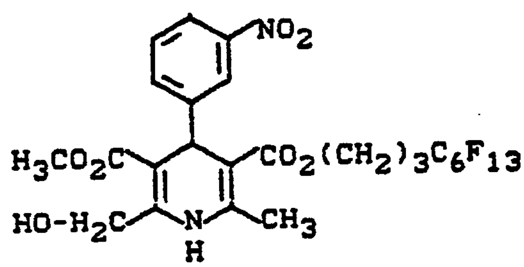

Analog Beispiel 51 wurde durch Solvolyse des 1,4-Dihydro-2-acetoxymethyl-6-methyl-4-(3-nitrophenyl)-pyridin-3,5-dicarbonsäure-3-methyl-5-(3-perfluorhexyl-propyl)-esters (Beispiel 42) der 1,4-Dihydro-2-hydroxymethyl-6-methyl-4-(3-nitrophenyl)-pyridin-3,5-dicarbonsäure-3-methyl-5-(3-perfluorhexyl-propyl)-ester vom Fp.: 106° C erhalten.
Ausbeute: 56 % der Theorie.

**Patentansprüche**

1. 1,4-Dihydropyridine der allgemeinen Formel I

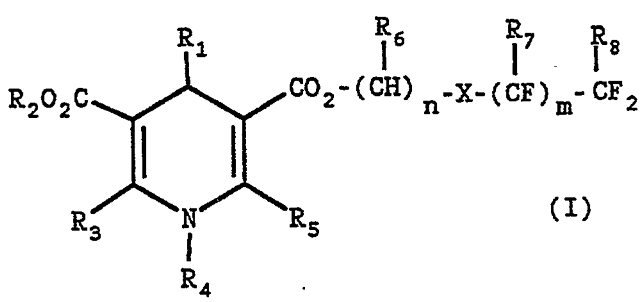

(I)

in welcher
$R_1$ für Phenyl oder Pyridyl steht, welche gegebenenfalls durch 1 oder 2 gleiche oder verschiedene

Substituenten aus der Gruppe Chlor, Trifluormethyl, Nitro oder Cyano substituiert sind, oder für Benzoxadiazolyl steht,

$R_2$ einen geradkettigen, verzweigten oder cyclischen Kohlenwasserstoffrest mit bis zu 8 Kohlenstoffatomen darstellt, der gegebenenfalls durch 1 Sauerstoffatom in der Kette unterbrochen ist und/oder der gegebenenfalls substituiert ist durch Fluor, Cyano, Acetoxy, Phenyl, Phenoxy, α-, β- oder γ-Pyridyl oder durch eine Aminogruppe, wobei diese Aminogruppe 2 gleiche oder verschiedene Substituenten aus der Gruppe Alkyl mit bis zu 4 C-Atomen oder Benzyl trägt,

$R_3$ und $R_5$ gleich oder verschieden sind und jeweils für einen geradkettigen oder cyclischen Alkylrest mit bis zu 6 Kohlenstoffatomen stehen, oder

einer der Substituenten $R_3$ oder $R_5$ einen Alkylrest mit bis zu 4 Kohlenstoffatomen darstellt, der durch Acetoxy, Hydroxy, Phthalimido, Amino, Phthalimidoethoxy oder Aminoethoxy substituiert ist, oder

einer der Substituenten $R_3$ oder $R_5$ für die Formyl- oder Nitrilgruppe steht,

$R_4$ für Wasserstoff oder den Morpholinoethylrest steht,

$R_6$ Wasserstoff bedeutet,

$R_7$ und $R_8$ jeweils ein Fluoratom darstellen,

n >/=1 und m >/=0 ist, wobei die Summe von n und m für eine ganze Zahl von 5 bis 15 steht und

X eine Einfachbindung oder die Gruppe $-N(CH_3)O_2S-$ darstellt,

in Form ihrer racemischen Gemische und ihrer Enantiomeren.

2. Verfahren zur Herstellung von Verbindungen der allgemeinen Formel I

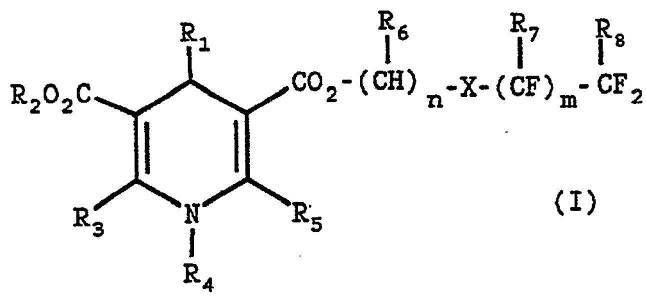

(I)

in welcher

$R_1$ für Phenyl oder Pyridyl steht, welche gegebenenfalls durch 1 oder 2 gleiche oder verschiedene Substituenten aus der Gruppe Chlor, Trifluormethyl, Nitro oder Cyano substituiert sind, oder für Benzoxadiazolyl steht,

$R_2$ einen geradkettigen, verzweigten oder cyclischen Kohlenwasserstoffrest mit bis zu 8 Kohlenstoffatomen darstellt, der gegebenenfalls durch 1 Sauerstoffatom in der Kette unterbrochen ist und/oder der gegebenenfalls substituiert ist durch Fluor, Cyano, Acetoxy, Phenyl, Phenoxy, α-, β- oder γ-Pyridyl oder durch eine Aminogruppe, wobei diese Aminogruppe 2 gleiche oder verschiedene Substituenten aus der Gruppe Alkyl mit bis zu 4 C-Atomen oder Benzyl trägt,

$R_3$ und $R_5$ gleich oder verschieden sind und jeweils für einen geradkettigen oder cyclischen Alkylrest mit bis zu 6 Kohlenstoffatomen stehen, oder

einer der Substituenten $R_3$ oder $R_5$ einen Alkylrest mit bis zu 4 Kohlenstoffatomen darstellt, der durch Acetoxy, Hydroxy, Phthalimido, Amino, Phthalimidoethoxy oder Aminoethoxy substituiert ist, oder

einer der Substituenten $R_3$ oder $R_5$ für die Formyl- oder Nitrilgruppe steht,

$R_4$ für Wasserstoff oder den Morpholinoethylrest steht,

$R_6$ Wasserstoff bedeutet,

$R_7$ und $R_8$ jeweils ein Fluoratom darstellen,

n >/=1 und m >/=0 ist, wobei die Summe von n und m für eine ganze Zahl von 5 bis 15 steht und

X eine Einfachbindung oder die Gruppe $-N(CH_3)O_2S-$ darstellt, dadurch gekennzeichnet, daß man

A) Yliden-β-ketoester der allgemeinen Formel II

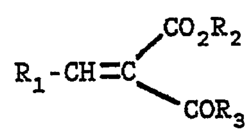

(II)

in welcher

$R_1$, $R_2$ und $R_3$ die oben angegebene Bedeutung haben, mit Enaminocarbonsäureestern der allgemeinen Formel III

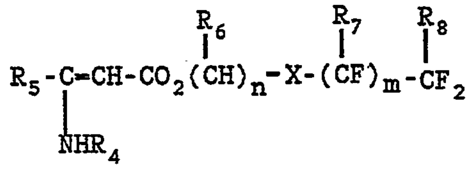

(III)

in welcher

$R_4$, $R_5$, $R_6$, $R_7$, $R_8$, X, n und m die oben angegebene Bedeutung haben, gegebenenfalls in Gegenwart inerter organischer Lösungsmittel umsetzt, oder

B) Yliden-β-ketoester der allgemeinen Formel II

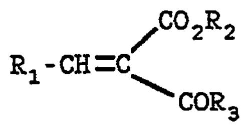

(II)

in welcher

$R_1$, $R_2$ und $R_3$ die oben angegebene Bedeutung haben, mit β-Ketocarbonsäureestern der allgemeinen Formel IV und Aminen der allgemeinen Formel V

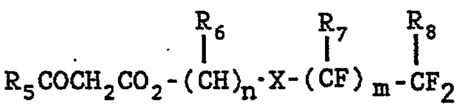

$R_4-NH_2$

(IV) (V)

in welchen

$R_4$, $R_5$, $R_6$, $R_7$, $R_8$, X und n und m die oben angegebene Bedeutung haben, gegebenenfalls in Gegenwart inerter organischer Lösungsmittel umsetzt, oder

C) Yliden-β-ketoester der allgemeinen Formel VI

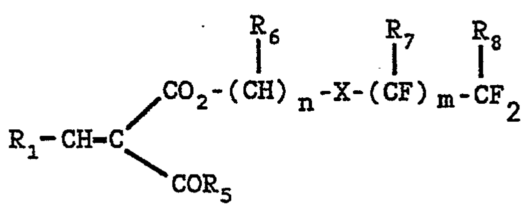

(VI)

in welcher

$R_1$, $R_5$, $R_6$, $R_7$, $R_8$, X, n und m die oben angegebene Bedeutung haben, mit Enaminocarbonsäureestern der allgemeinen Formel VII

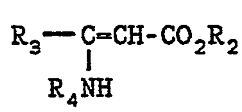

(VII)

in welcher

$R_2$, $R_3$ und $R_4$ die oben angegebene Bedeutung haben, gegebenenfalls in Gegenwart inerter organischer Lösungsmittel umsetzt, oder

D) Yliden-β-ketoester der allgemeinen Formel VI,

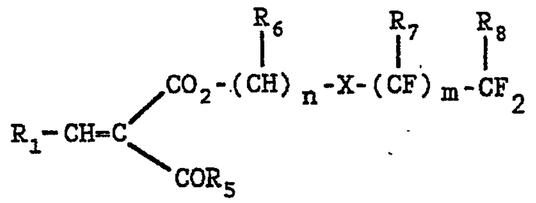 (VI)

in welcher
$R_1$, $R_5$, $R_6$, $R_7$, $R_8$, X, n und m die oben angegebene Bedeutung haben, mit β-Ketocarbonsäureestern der allgemeinen Formel VIII und Aminen der allgemeinen Formel V

$R_3COCH_2CO_2R_2$ (VIII) $R_4\text{-}NH_2$ (V)

in welchen
$R_2$, $R_3$ und $R_4$ die oben angegebene Bedeutung haben, gegebenenfalls in Gegenwart inerter organischer Lösungsmittel umsetzt, oder
E) Aldehyde der allgemeinen Formel IX

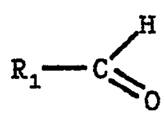 (IX)

in welcher
$R_1$ die oben angegebene Bedeutung hat, mit Enaminocarbonsäureestern der allgemeinen Formel II und β-Ketocarbonsäureestern der allgemeinen Formel VIII

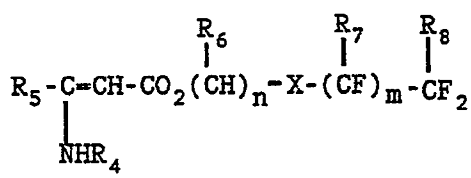 (III)

$R_3COCH_2CO_2R_2$ (VIII)

in welchen
$R_2$, $R_3$, $R_4$, $R_5$, $R_6$, $R_7$, $R_8$, X, n und m die oben angegebene Bedeutung haben, gegebenenfalls in Gegenwart inerter organischer Lösungsmittel zur Reaktion bringt, oder
F) Aldehyde der allgemeinen Formel IX

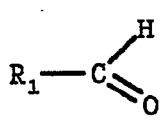 (IX)

in welcher
$R_1$ die oben angegebene Bedeutung hat, mit Enaminocarbonsäureestern der allgemeinen Formel VII und β-Ketocarbonsäureestern der allgemeinen Formel IV

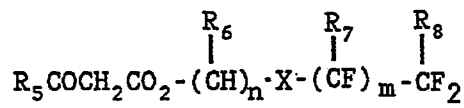

(IV)

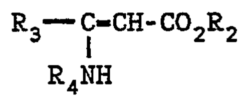 (VII)

in welchen
$R_2$, $R_3$, $R_4$, $R_5$, $R_6$, $R_7$, $R_8$, X, n und m die oben angegebene Bedeutung haben, gegebenenfalls in Gegenwart inerter organischer Lösungsmittel umsetzt, oder

G) die 1,4-Dihydropyridinmonocarbonsäuren der allgemeinen Formel X bzw. XI

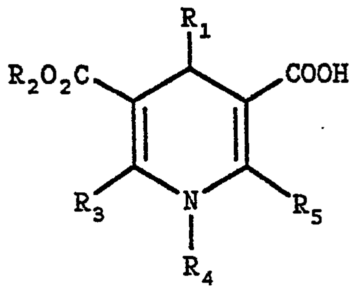

(X)

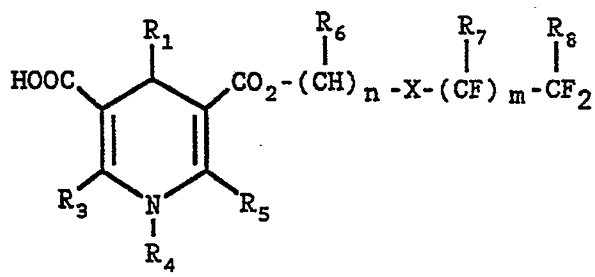

(XI)

in welchen
$R_1$, $R_2$, $R_3$, $R_4$, $R_5$, $R_6$, $R_7$, $R_8$, X, n und m die oben angegebene Bedeutung haben, nach üblichen Methoden der Veresterung von Carbonsäuren (z.B. über das Säurechlorid oder -imidazolid oder in Gegenwart von Dicyclohexylcarbodiimid) mit Alkoholen der allgemeinen Formel XII bzw. XIII

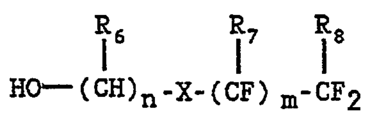

(XII)

$R_2$—OH

(XIII)

in welchen
$R_2$, $R_6$, $R_7$, $R_8$, X, n und m die oben angegebene Bedeutung haben, verestert.

3. Verfahren zur Herstellung von optisch aktiven Verbindungen der allgemeinen Formel I gemäß Anspruch 2, dadurch gekennzeichnet, daß man optisch aktive enantiomerenreine Dihydropyridinmonocarbonsäuren der allgemeinen Formel X oder XI gemäß Anspruch 2, in denen die jeweiligen $C_4$-Kohlenstoffatome des Dihydropyridinringes die alleinigen Chiralitätszentren darstellen, mit achiralen Alkoholen der Formeln X oder XIII gemäß Anspruch 2 verestert.

4. Verfahren zur Herstellung von Verbindungen der allgemeinen Formel I gemäß Anspruch 2, in welcher die Substituenten $R_1$, $R_2$, $R_4$, $R_6$, $R_7$, $R_8$, n, m und X die im Anspruch 2 angegebene Bedeutung haben und

$R_3$ oder $R_5$ für Formyl, Nitril oder Alkyl, welches gegebenenfalls durch Hydroxy, Amino oder Aminoalkoxy substituiert ist, stehen, dadurch gekennzeichnet, daß man gemäß Anspruch 2 geeignete Dihydropyridine synthetisiert und diese in Folgereaktionen weiter umsetzt, wobei für den Fall

a) $R_3$ oder $R_5$ für die Formylgruppe oder die Nitrilgruppe steht, diese durch saure Hydrolyse des entsprechenden Dialkoxymethylrestes erhalten wird, wobei die Formylverbindungen anschließend durch Reaktion mit Hydroxylamin und anschließende Dehydratisierung des entsprechenden Oxims nach üblichen Methoden in die entsprechenden Nitrilverbindungen umgesetzt werden,

b) daß $R_3$ oder $R_5$ für Hydroxyalkyl stehen, die entsprechenden erfindungsgemäßen Acyloxiderivate sauer- oder alkalisch hydrolisiert werden,

c) daß $R_3$ oder $R_5$ für Aminoalkyl oder Aminoalkoxyalkyl stehen die entsprechend nach dem Verfahren gemäß Anspruch 5 hergestellten Phthalimidoverbindungen hydrazinolysiert oder die Phthalimidoalkoxyverbindungen mit Hydrazin umgesetzt werden.

5. Verbindungen der allgemeinen Formel I gemäß Anspruch 1 zur Verwendung bei der Bekämpfung von Erkrankungen.

6. Arzneimittel enthaltend mindestens eine Verbindung der allgemeinen Formel I gemäß Anspruch 1.

7. Verfahren zur Herstellung von Arzneimitteln, dadurch gekennzeichnet, daß man mindestens eine Verbindung der allgemeinen Formel I gemäß Anspruch 2 gegebenenfalls unter Einsatz von Hilfs- und Trägerstoffen in eine geeignete Applikationsform überführt.

8. Verwendung von Verbindungen der allgemeinen Formel I gemäß Anspruch 1 bei der Herstellung von Kreislaufmitteln.

**Claims**

1. 1,4-Dihydropyridines of the general formula I

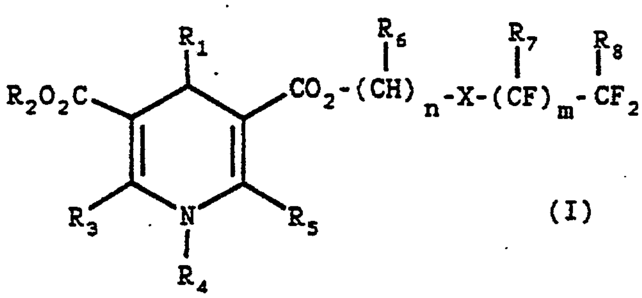

(I)

in which

$R_1$ represents phenyl or pyridyl, which are optionally substituted by 1 or 2 identical or different substituents from the group comprising chlorine, trifluoromethyl, nitro or cyano, or represents benzoxadiazolyl,

$R_2$ represents a straight-chain, branched or cyclic hydrocarbon radical having up to 8 carbon atoms which is optionally interrupted by 1 oxygen atom in the chain and/or is optionally substituted by fluorine, cyano, acetoxy, phenyl, phenoxy, α-, β- or γ-pyridyl or by a amino group, this amino group carrying 2 identical or different substituents from the group comprising alkyl having up to 4 C atoms,

$R_3$ and $R_5$ are identical or different and each represents a straight-chain or cyclic alkyl radical having up to 6 carbon atoms, or one of the substituents $R_3$ or $R_5$ represents an alkyl radical having up to 4 carbon atoms, which is substituted by acetoxy, hydroxyl, phthalimido, amino, phthalimidoethoxy or aminoethoxy, or one of the substituents

$R_3$ or $R_5$ represents the formyl or nitrile group,

$R_4$ represents hydrogen or the morpholinoethyl radical,

$R_6$ represents hydrogen

$R_7$ and $R_8$ each represent a fluorine atom,

$n \geq 1$ and

$m \geq 0$, and the sum of n and m represents an integer from 5 to 15, and

X represents a single bond or the group $-N(CH_3)O_2S-$, in the form of their racemie mixtures and their enantiomers.

2. Process for the preparation of compounds of the general formula I

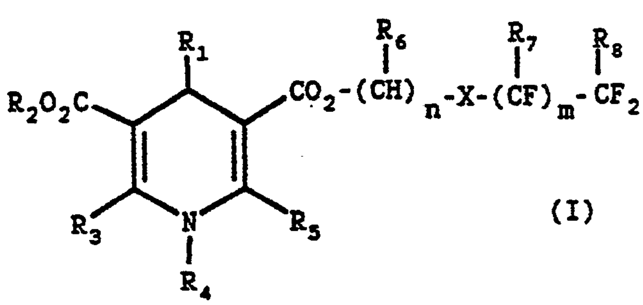

(I)

in which

$R_1$ represents phenyl or pyridyl, which are optionally substituted by 1 or 2 identical or different substituents from the group comprising chlorine, trifluoromethyl, nitro or cyano, or represents benzoxadiazolyl,

$R_2$ represents a straight-chain, branched or cyclic hydrocarbon radical having up to 8 carbon atoms which is optionally interrupted by 1 oxygen atom in the chain and/or is optionally substituted by fluorine, cyano, acetoxy, phenyl, phenoxy, α-, β- or γ-pyridyl or by an amino group, this amino group carrying 2 identical or different substituents from the group comprising alkyl having up to 4 C atoms,

$R_3$ and $R_5$ are identical or different and each represents, a straight-chain or cyclic alkyl radical having up to 6 carbon atoms, or one of the substituents $R_3$ or $R_5$ represents an alkyl radical having up to 4 carbon atoms, which is substituted by acetoxy, hydroxyl, phthalimido, amino phthalimido-ethoxy or aminoethoxy, or one of the substituents,

$R_3$ or $R_5$ represents the formyl of nitrile group,

$R_4$ represents hydrogen or the morpholinoethyl radical,

$R_6$ represents hydrogen

$R_7$ and $R_8$ each represent a fluorine atom,

$n \geq 1$ and

$m \geq 0$, and the sum of n and m represents an integer from 5 to 15, and

X represents a single bond or the group $-N(CH_3)O_2S-$, in the form of their racemil mixtures and their enantiomers, characterized in that,

A) ylidene-β-keto esters of the general formula II

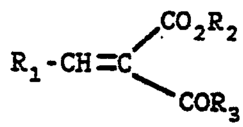 (II)

in which

$R_1$, $R_2$ and $R_3$ have the abovementioned meaning, are reacted with enaminocarboxylic acid esters of the general formula III

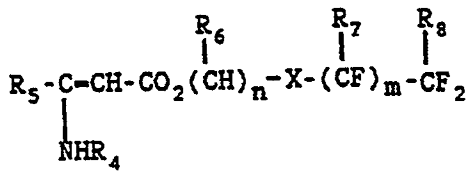 (III)

in which

$R_4$, $R_5$, $R_6$, $R_7$, $R_8$, X, n and m have the abovementioned meaning,

if appropriate in the presence of inert organic solvents,

or

B) ylidene-β-keto esters of the general formula II

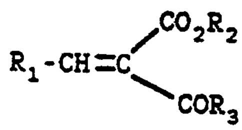 (II)

in which

$R_1$, $R_2$ and $R_3$ have the abovementioned meaning,

are reacted with β-ketocarboxylic acid esters of the general formula IV and amines of the general formula V

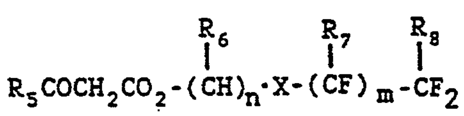

(IV)

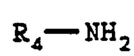

(V)

in which

$R_4$, $R_5$, $R_6$, $R_7$, $R_8$, X, n and m have the abovementioned meaning

if appropriate in the presence of inert organic solvents, or

C) ylidene-β-keto esters of the general formula VI

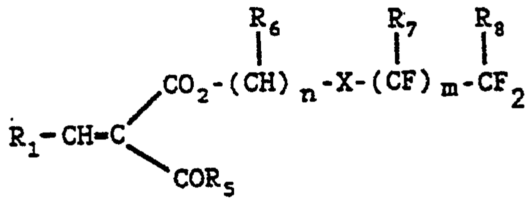

(VI)

in which
$R_1$, $R_5$, $R_6$, $R_7$, $R_8$, X, n and m have the abovementioned meaning,
are reacted with enaminocarboxylic acid esters of the general formula VII

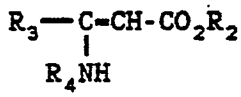

(VII)

in which
$R_2$, $R_3$ and $R_4$ have the abovementioned meaning,
if appropriate in the presence of inert organic solvents,
or
D) ylidene-β-keto esters of the general formula VI

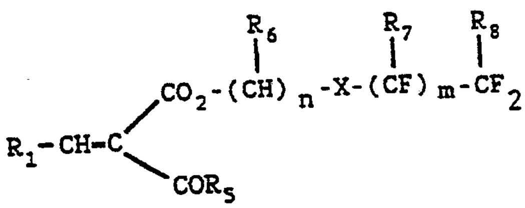

(VI)

in which
$R_1$, $R_5$, $R_6$, $R_7$, $R_8$, X, n and m have the abovementioned meaning,
are reacted with β-ketocarboxylic acid esters of the general formula VIII and amines of the general formula V

$R_3COCH_2CO_2R_2$ (VIII) $\qquad$ $R_4$-$NH_2$ (V)

in which
$R_2$, $R_3$ and $R_4$ have the abovementioned meaning,
if appropriate in the presence of inert organic solvents, or
E) aldehydes of the general formula IX

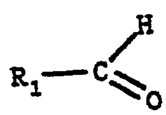

(IX)

in which
$R_1$ has the abovementioned meaning, are reacted with enaminocarboxylic acid esters of the general formula III and β-ketocarboxylic acid esters of the general formula VIII

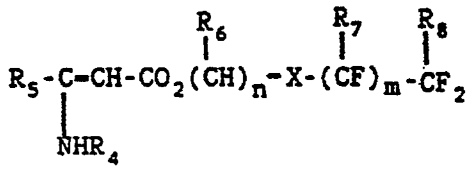

(III)

$R_3COCH_2CO_2R_2$ (VIII)

in which
$R_2$, $R_3$, $R_4$, $R_5$, $R_6$, $R_7$, $R_8$, X, n and m have the abovementioned meaning,
if appropriate in the presence of inert organic solvents, or
F) aldehydes of the general formula IX

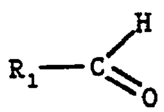

(IX)

in which
$R_1$ has the abovementioned meaning,
are reacted with enaminocarboxylic acid esters of the general formula VII and β-ketocarboxylic acid esters of the general fcrmula IV

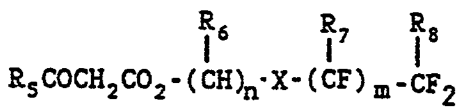

(IV)

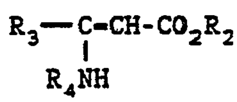

(VII)

in which
$R_2$, $R_3$, $R_4$, $R_5$, $R_6$, $R_7$, $R_8$, X, n and m have the abovementioned meaning,
if appropriate in the presence of inert organic solvents, or
G) the 1,4-dihydropyridinemonocarboxylic acids of the general fcrmulae X or XI

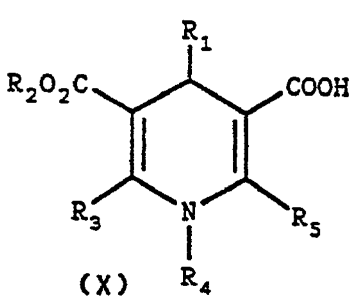

(X)

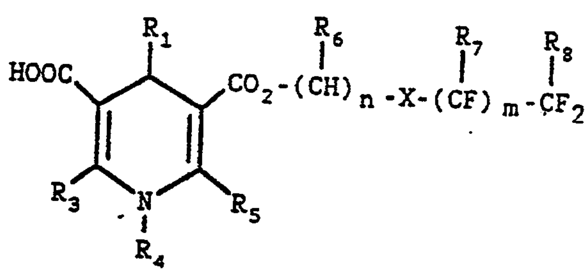

(XI)

in which
$R_1$, $R_2$, $R_3$, $R_4$, $R_5$, $R_6$, $R_7$, $R_8$, X, n and m have the abovementioned meaning,
are esterified with alcohols of the general formula XII or XIII

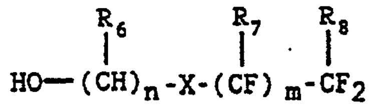

(XII)

$R_2$—OH

(XIII)

in which
$R_2$, $R_6$, $R_7$, $R_8$, X, n and m have the abovementioned meaning,
by the customary methods of esterification of carboxylic acids (for example via the acid chloride or imidazolide or in the presence of dicyclohexylcarbodiimide).

3. Process for the preparation of optically active compounds of the general formula I according to Claim 2, characterized in that optically active enantiomerically pure dihydropyridinemonocarboxylic acids of the general formula X or XI according to Claim 2, in which the particular $C_4$ carbon atoms of the dihydropyridine ring represent the sole chirality centres, are esterified with achiral alcohols of the formulae (X) or (XIII) according to Claim 2.

4. Process for the preparation of compounds of the general formula I according to Claim 2, in which the substituents $R_1$, $R_2$, $R_4$, $R_6$, $R_7$, $R_8$, n, m and X have the meaning given in Claim 2 and
$R_3$ or $R_5$ represents formyl, nitrile or alkyl which is optionally substituted by hydroxyl, amino or aminoalkoxy, characterized in that suitable dihydropyridines are synthesized by process variants A to G according to Claim 2 and these are further reacted in subsequent reactions, and in the case

a) where $R_3$ or $R_5$ represents the formyl group or the nitrile group, this is obtained by acid hydrolysis of the corresponding dialkoxymethyl radical, the formyl compounds subsequently being converted into the corresponding nitrile compounds by reaction with hydroxylamine and subsequent dehydration of the corresponding oxime by customary methods,

b) where $R_3$ or $R_5$ represents hydroxylalkyl, the corresponding acyloxy derivatives according to the invention are hydrolysed under acid or alkaline conditions, and

c) where $R_3$ or $R_5$ represents aminoalkyl or aminoalkoxyalkyl, the phthalimido compounds correspondingly prepared by the process according to Claim 2 are hydrazinolysed, or the phthalimidoalkoxy compounds are reacted with hydrazine.

5. Compounds of the general formula I according to Claim 1 for use in combating diseases.

6. Medicaments containing at least one compound of the general formula I according to Claim 1.

7. Process for the preparation of medicaments, characterized in that at least one compound of the general formula I according to Claim 2 is converted into a suitable administration form, if appropriate using auxiliaries and excipients.

8. Use of compounds of the general formula I according to Claim 1 in the preparation of cardiovascular agents.

**Revendications**

1. 1,4-dihydropyridines de formule générale I

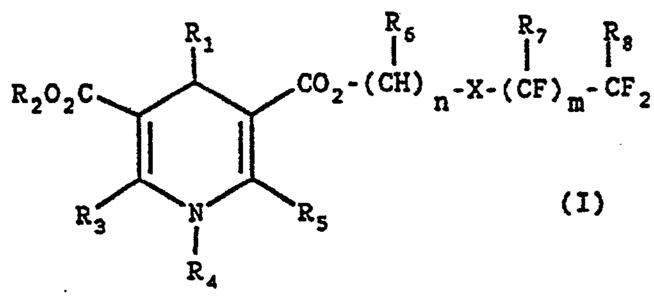

(I)

dans laquelle
$R_1$ représente un groupe phényle ou pyridyle qui porte éventuellement un ou deux substituants identiques ou différents choisis parmi le chlore, les groupes trifluorométhyle, nitro ou cyano; ou un groupe benzoxadiazolyle,
$R_2$ représente un radical hydrocarboné à chaîne droite, ramifiée ou cyclique, contenant jusqu'à 8 atomes de carbone, éventuellement interrompu dans la chaîne par un atome d'oxygène et/ou éventuellement substitué par le fluor, des groupes cyano, acétoxy, phényle, phénoxy, α-, β- ou γ-pyridyle ou par un

groupe amino, ce dernier portant deux substituants identiques ou différents choisis parmi les groupes alkyle en $C_1$–$C_4$ et les groupes benzyle,

$R_3$ et $R_5$, ayant des significations identiques ou différentes, représentent chacun un groupe alkyle à chaîne droite ou cyclique contenant jusqu'à 6 atomes de carbone, ou bien

l'un des symboles $R_3$ ou $R_5$ représente un groupe alkyle contenant jusqu'à 4 atomes de carbone substitué par des groupes acétoxy, hydroxy, phtalimido, amino, phtalimidoéthoxy ou aminoéthoxy, ou bien

l'un des symboles $R_3$ ou $R_5$ représente un groupe formyle ou nitrile,

$R_4$ représente l'hydrogène ou le groupe morpholinoéthyle,

$R_6$ représente l'hydrogène,

$R_7$ et $R_8$ représentent chacun un atome de fluor,

n est supérieure ou égal à 1 et m est supérieur ou égal à 0, la somme de n et m étant un nombre entier de 5 à 15, et

X représente une liaison simple ou le groupe $-N-(CH_3)O_2S-$, à l'état de mélanges racémiques et d'énantiomères.

2. Procédé de préparation des composés de formule générale I

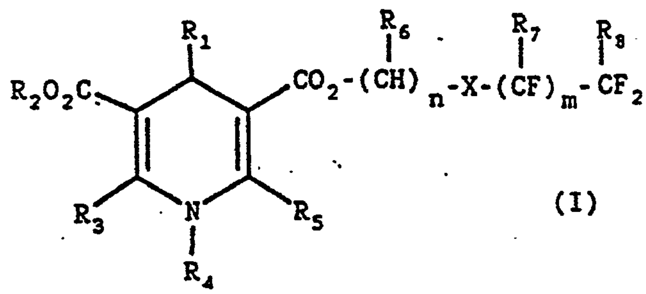

(I)

dans laquelle

$R_1$ représente un groupe phényle ou pyridyle qui porte éventuellement un ou deux substituants identiques ou différents choisis parmi le chlore, les groupes trifluorométhyle, nitro ou cyano; ou bien un groupe benzoxiadiazolyle,

$R_2$ représente un radical hydrocarboné à chaîne droite, ramifiée ou cyclique contenant jusqu'à 8 atomes de carbone, éventuellement interrompu dans la chaîne par un atome d'oxygène et/ou éventuellement substitué par le fluor, des groupes cyano, acétoxy, phényle, phénoxy, α-, β- ou γ-pyridyle ou par un groupe amino portant lui-même deux substituants identiques ou différents choisis parmi les groupes alkyle en $C_1$–$C_4$ et benzyle,

$R_3$ et $R_5$, ayant des significations identiques ou différents, représentent chacun un groupe alkyle à chaîne droite ou cyclique contenant jusqu'à 6 atomes de carbone, ou bien

l'un des symboles $R_3$ ou $R_5$ représente un groupe alkyle contenant jusqu'à 4 atomes de carbone, substitué par des groupes acétoxy, hydroxy, phtalimido, amino, phtalimidoéthoxy ou aminoéthoxy, ou bien

l'un des symboles $R_3$ ou $R_5$ représente un groupe formyle ou nitrile,

$R_4$ représente l'hydrogène ou le groupe morpholinoéthyle,

$R_6$ représente l'hydrogène,

$R_7$ et $R_8$ représentent chacun un atome de fluor,

n est supérieur ou égal à 1 et m est supérieur ou égal à 0, la somme de n et m étant un nombre entier de 5 à 15, et

X représente une liaison simple ou le groupe $-N-(CH_3)O_2S-$, caractérisé en ce que:

A) on fait réagir des ylidène-β-cétoesters de formule générale II

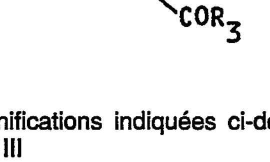

(II)

dans laquelle

$R_1$, $R_2$ et $R_3$ ont les significations indiquées ci-dessus, avec des esters d'acides énaminocarboxyliques de formule générale III

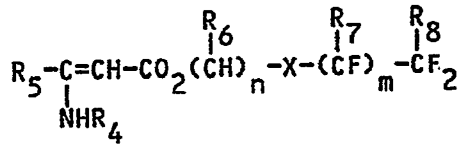

(III)

dans laquelle
$R_4$, $R_5$, $R_6$, $R_7$, $R_8$, X, n et m ont les significations indiquées ci-dessus, éventuellement en présence de solvants organiques inertes, ou bien
B) on fait réagir des ylidène-β-cétoesters de formule générale II

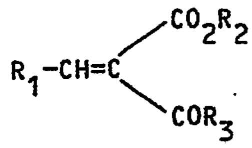

(II)

dans laquelle
$R_1$, $R_2$ et $R_3$ ont les significations indiquées ci-dessus, avec des esters d'acides β-cétocarboxyliques de formule générale IV et des amines de formule générale V

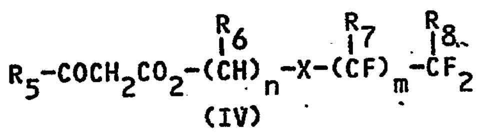

(IV)

$R_4-NH_2$

(V)

dans lesquelles
$R_4$, $R_5$, $R_6$, $R_7$, $R_8$, X, n et m ont les significations indiquées ci-dessus, éventuellement en présence de solvants organiques inertes, ou bien
C) on fait réagir des ylidène-β-cétoesters de formule générale VI

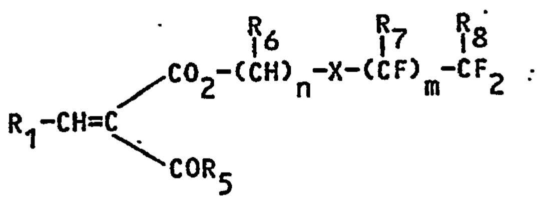

(VI)

dans laquelle
$R_1$, $R_5$, $R_6$, $R_7$, $R_8$, X, n et m ont les significations indiquées ci-dessus, avec des esters d'acides énaminocarboxyliques de formule générale VI

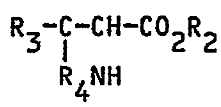

(VII)

dans laquelle
$R_2$, $R_3$ et $R_4$ ont les significations indiquées ci-dessus, éventuellement en présence de solvants organiques inertes, ou bien
D) on fait réagir des ylidène-β-cétoesters de formule générale VI

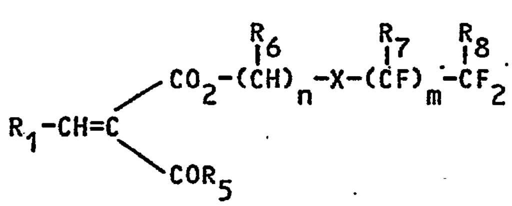

(VI)

dans laquelle
$R_1$, $R_5$, $R_6$, $R_7$, $R_8$, X, n et m ont les significations indiquées ci-dessus, avec des esters d'acides β-cétocarboxyliques de formule générale VII et des amines de formule générale V

$R_3COCH_2CO_2R_2$ (VIII) $R_4-NH_2$ (V)

dans lesquelles
$R_2$, $R_3$ et $R_4$ ont les significations indiquées ci-dessus, éventuellement en présence de solvants organiques inertes, ou bien
E) on fait réagir des aldéhydes de formule générale IX

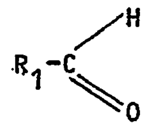

(IX)

dans laquelle
$R_1$ a les significations indiquées ci-dessus,
avec des esters d'acides énaminocarboxyliques de formule générale II et des esters d'acides β-cétocarboxyliques de formule générale VIII

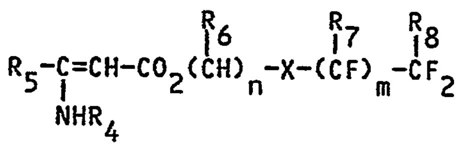

(III)

$R_3COCH_2CO_2R_2$

(VIII)

dans lesquelles
$R_2$, $R_3$, $R_4$, $R_5$, $R_6$, $R_7$, $R_8$, X, n et m ont les significations indiquées ci-dessus, éventuellement en présence de solvants organiques inertes, ou bien
F) on fait réagir des aldéhydes de formule générale IX

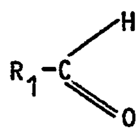

(IX)

dans laquelle
$R_1$ a les significations indiquées ci-dessus,
avec des esters d'acides énaminocarboxyliques de formule générale VII et des esters d'acides β-cétocarboxyliques de formule générale IV

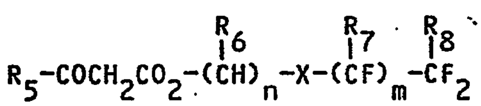

(IV)

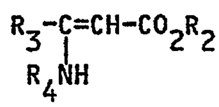

(VII)

dans lesquelles
$R_2$, $R_3$, $R_4$, $R_5$, $R_6$, $R_7$, $R_8$, X, n et m ont les significations indiquées ci-dessus, éventuellement en présence de solvants organiques inertes, ou bien
G) on estérifie les acides 1,4-dihydropyridine-monocarboxyliques de formules générales respectives X et XI

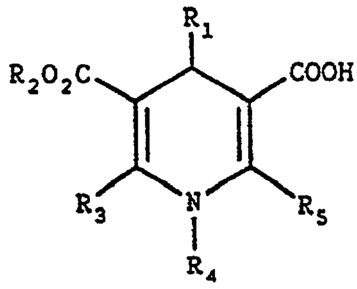

(X)

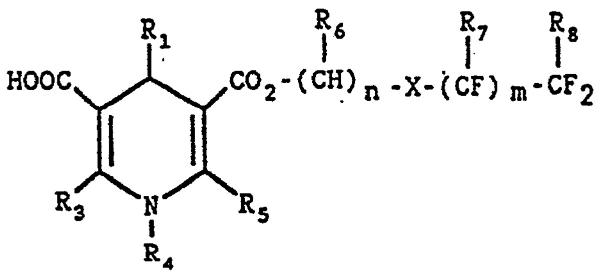

(XI)

dans lesquelles

$R_1$, $R_2$, $R_3$, $R_4$, $R_5$, $R_6$, $R_7$, $R_8$, X, n et m ont les significations indiquées ci-dessus,

par les modes opératoires usuels d'estérification des acides carboxyliques (par exemple en passant par le chlorure ou l'imidazolide d'acide ou en présence de dicyclohexylcarbodiimide) par des alcools de formules générales respectives XII et XIII

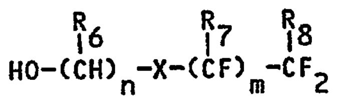

(XII)

$R_2$-OH

(XIII)

dans lesquelles

$R_2$, $R_6$, $R_7$, $R_8$, X, n et m ont les significations indiquées ci-dessus.

3. Procédé de préparation de composés de formule générale I à l'état d'isomères optiques, selon la revendication 2, caractérisé en ce que l'on estérifie des acides dihydropyridine-monocarboxyliques de formule générale X ou XI selon la revendication 2 à l'état d'isomères optiques purs et dans lesquels les atomes de carbone $C_4$ du cycle de dihydropyridine constituent les uniques centres de chiralité, par des alcools non chiraux de formule X ou XIII selon la revendication 2.

4. Procédé de préparation de composés de formule générale I selon la revendication 2, dans lesquels les symboles $R_1$, $R_2$, $R_4$, $R_6$, $R_7$, $R_8$, n, m et X ont les significations indiquées dans la revendication 2, et

$R_3$ ou $R_5$ représente un groupe formyle, nitrile ou alkyle éventuellement substitué par des groupes hydroxy, amino ou aminoalcoxy, caractérisé en ce que l'on prépare, par les variantes opératoires A) à G) de la revendication 2, des dihydropyridines appropriées qu'on convertit ensuite dans des réactions subséquentes, et plus précisément

a) dans le cas où $R_3$ ou $R_5$ représente un groupe formyle ou un groupe nitrile, on obtient ces groupes par hydrolyse acide du groupe correspondant dialcoxyméthyle, les composés formylés étant ensuite convertis en les nitriles correspondants par réaction avec l'hydroxylamine suivie de la déshydratation de l'oxime correspondante par des modes opératoires usuels,

b) dans le cas où $R_3$ ou $R_5$ représente un groupe hydroxyalkyle, ou soumet les dérivés correspondants acyloxylés selon l'invention à une hydrolyse acide ou alcaline,

c) dans le cas où $R_3$ ou $R_5$ représente un groupe aminoalkyle ou aminoalcoxyalkyle, on soumet les dérivés phtalimidés correspondants préparés par le procédé de la revendication 5 à hydrazinolyse ou bien on fait réagir les dérivés phtalimido-alcoxylés avec l'hydrazine.

5. Composés de formule générale I selon la revendication 1 pour l'utilisation dans le traitement de maladies.

6. Médicament contenant au moins un composé de formule générale I selon la revendication 1.

7. Procédé de préparation de médicaments, caractérisé en ce que l'on met au moins un composé de formule générale I selon la revendication 2, éventuellement avec utilisation de produits auxiliaires et véhicules, sous une forme d'administration appropriée.

8. Utilisation des composés de formule générale I selon la revendication 1 pour la préparation d'agents agissant sur la circulation.